# EUROPÄISCHE PATENTSCHRIFT

(11) **EP 3 649 179 B1**
(45) Veröffentlichungstag und Bekanntmachung des Hinweises auf die Patenterteilung: **11.08.2021**
(21) Anmeldenummer: 18733301.8
(22) Anmeldetag: 29.06.2018
(51) Int. Cl.: C08G 64/20, C07C 263/10, B01J 19/00

(54) **PRODUKTIONSANLAGE ZUR HERSTELLUNG EINES CHEMISCHEN PRODUKTS DURCH UMSETZUNG H-FUNKTIONELLER REAKTANTEN MIT PHOSGEN UND VERFAHREN ZUM BETREIBEN DERSELBEN**
PRODUCTION LINE FOR MANUFACTURE OF A CHEMICAL PRODUCT BY REACTING H FUNCTIONAL REACTANTS WITH PHOSGENE AND METHOD FOR OPERATING THE SAME
INSTALLATION DE PRODUCTION DESTINÉE À FABRIQUER UN PRODUIT CHIMIQUE PAR MISE EN RÉACTION DE RÉACTIFS FONCTIONNELS H À L'AIDE DE PHOSGÈNE ET SON PROCÉDÉ DE FONCTIONNEMENT

(30) Priorität: 03.07.2017 EP 17179378
(43) Veröffentlichungstag der Anmeldung: 13.05.2020
(73) Patentinhaber: Covestro Intellectual Property GmbH & Co. KG, 51373 Leverkusen (DE)
(72) Erfinder: SPRIEWALD, Jürgen, 77586 Seabrook (US); KNAUF, Thomas, 41542 Dormagen (DE); MANZEL, Dirk, 47447 Moers (DE); PLATHEN, Peter, 47829 Krefeld (DE)
(74) Vertreter: Levpat
(86) Internationale Anmeldenummer: PCT/EP2018/067569
(87) Internationale Veröffentlichungsnummer: WO 2019/007834

(56) Entgegenhaltungen:
- WO-A1-2015/197522
- WO-A1-2017/050776

## Beschreibung

Die Erfindung betrifft ein Verfahren zum Betreiben einer Produktionsanlage zur Herstellung eines chemischen Produkts (1) durch Umsetzung eines H-funktionellen Reaktanten (2) mit Phosgen (3) bei einer Produktionsunterbrechung unter Außerbetriebnahme mindestens eines Anlagenteils der Produktionsanlage, bei dem Sauerstoff-arme und Sauerstoff-reiche Phosgen-haltige Abgasströme getrennt voneinander in verschiedene Phosgenzersetzungsrichtungen und getrennt voneinander - an räumlich verschiedenen Stellen - in eine Verbrennungseinrichtung geleitet werden, wobei nicht außer Betrieb genommene Anlagenteile in Kreislauffahrweise betrieben werden. Die Erfindung betrifft ferner eine Produktionsanlage zur Herstellung eines chemischen Produkts durch Umsetzung H-funktioneller Reaktanten mit Phosgen, die dazu geeignet ist, mit dem erfindungsgemäßen Verfahren betrieben zu werden.

In vielen industriellen Produktionsprozessen, insbesondere Produktionsprozessen für chemische Produkte, fallen vom eigentlichen Produkt des Produktionsprozesses verschiedene gasförmige Verfahrensprodukte an, welche vor ihrer Entlassung als Abgase in die Umgebung aufgearbeitet werden müssen, um wirtschaftliche Verluste und Belastungen für die Umwelt zu minimieren. Solche Aufarbeitungsschritte können vielfältige Prozesse umfassen. Als Beispiele für solche Reinigungsoperationen seien die Wäsche, Absorption (z. B. von NOₓ), Adsorption, Kondensation und Filtration genannt. Es ist im Stand der Technik üblich, solche auf diese Weise vorgereinigten gasförmigen Verfahrensprodukte nach Abschluss aller zur Vorreinigung durchgeführten Reinigungsoperationen (ggf. auch schon vorher) zu vereinen und den so erhaltenen Abgasstrom einer gemeinsamen Abgasverbrennung zuzuführen, in welcher das Abgas so vollständig verbrannt wird, dass die Verbrennungsgase (erforderlichenfalls nach einer Nachreinigung wie bspw. einer Basenwäsche zur Bindung saurer Bestandteile) unter Einhaltung behördlicher Umweltauflagen in die Umgebung entlassen werden können.

Ein Beispiel für einen industriellen Produktionsprozess, in dem gasförmige Verfahrensprodukte anfallen, ist die Produktion von Isocyanaten durch Phosgenierung der korrespondierenden Amine. In der Phosgenierung fällt ein gasförmiger Verfahrensstrom umfassend das Koppelprodukt Chlorwasserstoff und nicht umgesetztes Phosgen an. Auch Reste des eingesetzten Lösungsmittels (sowie Inerte oder auch überschüssiges Kohlenmonoxid aus der Phosgenherstellung) sind regelmäßig hierin enthalten. In sich anschließenden Aufarbeitungsschritten können weitere gasförmige Verfahrensprodukte, die teilweise auch Lösungsmittel enthalten, anfallen. In diesem Zusammenhang spielt die Rückgewinnung von Lösungsmittel aus den verschiedenen gasförmigen Verfahrensprodukten, die im Prozess an unterschiedlichen Stellen anfallen, eine Rolle. Mit Prozessen zur Lösemittelrückgewinnung via Destillation in Isocyanat-Produktionsprozessen befassen sich z. B. EP 1 575 908 B2 und EP 1 575 906 B1.

Neben der Rückgewinnung von Lösungsmittel spielt auch die möglichst vollständige Rückgewinnung des Koppelprodukts Chlorwasserstoff und des überschüssigen Phosgens eine wichtige Rolle. WO 2004/056758 A1 beschreibt eine Nachreinigung von Chlorwasserstoff aus der Chlorwasserstoff/Phosgentrennung durch Adsorption von Verunreinigungen (Reste von Phosgen und Chlorbenzol) an Aktivkohle.

Nachdem alle Wertprodukte soweit wie möglich aus den anfallenden gasförmigen Verfahrensströmen zurückgewonnen wurden, verbleibt ein Abgasstrom, der noch Spurenanteile an Phosgen enthalten kann und daher vor Einleitung in die Abgasverbrennung eine Phosgenzersetzung durchläuft. Im Stand der Technik ist es üblich, die anfallenden Abgasströme zu vereinen und den so erhaltenen Gesamtabgasstrom einer gemeinsamen Phosgenzersetzung und anschließend der Verbrennung zuzuführen. Im Regelbetrieb ist diese Vorgehensweise im Allgemeinen völlig zufriedenstellend. Probleme treten jedoch auf, wenn die Produktion beispielsweise zur Durchführung von Reparaturarbeiten an einem Anlagenteil zeitweise unterbrochen werden muss. Sofern die Reparaturmaßnahme nicht zu lange dauert, ist es in einem solchen Fall zweckmäßig, nicht die gesamte Produktionsanlage außer Betrieb zu nehmen, sondern nur den von der Reparaturmaßahme betroffenen Anlagenteil, während die übrigen Anlagenteile in sog. Kreislauffahrweise weiter ohne Herstellung des angestrebten Zielprodukts betrieben werden. Diese Vorgehensweise ist in WO 2015/197522 A1 für die Produktion chemischer Produkte im Allgemeinen und in WO 2017/050776 A1 für die Produktion von Isocyanaten im Besonderen geschildert. In den in Kreislauffahrweise betriebenen Anlagenteilen können auch weiterhin Abgasströme anfallen, und sei es nur, weil die Leitungen, die diese Anlagenteile mit dem Abgassystem verbinden, vorzugsweise auch während der Kreislauffahrweise geöffnet bleiben, um der Anlage ein "Atmen" zu ermöglichen. Die hierbei anfallenden Abgasströme sind Sauerstoff-arm oder sogar Sauerstoff-frei. In dem zum Zwecke der Reparatur außer Betrieb genommenen Anlagenteil können aber Sauerstoff-reiche Abgasströme anfallen (z. B. infolge einer aus Gründen der Arbeitssicherheit betriebenen Absaugung), die aus Sicherheitsgründen ebenfalls die Phosgenzersetzung durchlaufen und verbrannt werden. Diese Sauerstoff-reichen Abgasströme können aber nicht ohne weiteres mit den zuvor genannten Sauerstoff-armen Abgasströmen vereinigt werden, sondern nur nach ausreichender Verdünnung z. B. mit Stickstoff, um die Bildung eines explosionsfähigen Gasgemisches zu verhindern. Diese Verdünnung verursacht aber zusätzliche Kosten, resultierend aus der Bereitstellung des Verdünnungsgases selbst, der Notwendigkeit, größere Volumenströme zu handhaben, aus Problemen in der Verbrennung infolge eines gegenüber dem Regelbetrieb vergrößerten Inertgasanteils und dergleichen mehr. Vermeiden lassen sich diese Probleme gemäß dem Stand der Technik nur bei Außerbetriebnahme der *gesamten* Produktionsanlage, weil in diesem Fall *ausschließlich* Sauerstoff-reiche Abgasströme anfallen. Hierdurch entfällt aber die Möglichkeit, die wirtschaftlichen Vorteile der oben genannten Kreislauffahrweise zu nutzen. Dieses am Beispiel der Isocyanat-Herstellung beschriebene Dilemma tritt bei der Herstellung von allen chemischen Produkten, die durch Phosgenierung von Verbindungen mit H-funktionellen Reaktanten gewonnen werden, auf.

Es wäre daher wünschenswert, ein Verfahren zum Betreiben von Produktionsanlagen zur Herstellung chemischer Produkte durch Umsetzung H-funktioneller Reaktanten mit Phosgen bei einer Produktionsunterbrechung zur Verfügung zu haben, das einen Ausweg aus dieser Problematik bietet.

Diesem Bedarf Rechnung tragend ist ein Gegenstand der vorliegenden Erfindung ein **Verfahren zum Betreiben einer Produktionsanlage** zur Herstellung eines chemischen Produkts (1) durch Umsetzung eines H-funktionellen Reaktanten (2) mit Phosgen (3) bei einer Produktionsunterbrechung, wobei die Produktionsanlage folgende Anlagenteile aufweist:
A) eine Reaktionsstrecke (1000) geeignet zur Umsetzung eines H-funktionellen Reaktanten (2) mit Phosgen (3), aufweisend:
   A.I) eine Mischzone (1100) zur Vermischung des H-funktionellen Reaktanten (2) und Phosgen (3) zu einem Reaktionsgemisch (50),
   A.II) eine mit der Mischzone (1100) verbundene Reaktionszone (1200) zur Umsetzung des in A.I) erhaltenen Reaktionsgemisches (50) unter Ausbildung einer Flüssigphase (60) enthaltend das chemische Produkt (1) sowie Phosgen (3) und eines Phosgen-haltigen Prozessabgasstroms (70);
B) eine mit der Reaktionsstrecke (1000) verbundene Aufarbeitungsstrecke (2000) aufweisend:
   B.I) eine Trenneinheit (2100-2500) zur Trennung der in A.II) erhaltenen Flüssigphase (60) in einen Phosgen-haltigen Prozessabgasstrom (170) und in eine Flüssigphase (100) enthaltend das chemische Produkt (1);
C) eine Abgasaufarbeitungsstrecke (3000) geeignet zur Aufarbeitung während der Herstellung des chemischen Produkts (1) und während der Produktionsunterbrechung anfallender Phosgen-haltiger Abgasströme, aufweisend eine erste Phosgenzersetzungseinrichtung (3011) und eine zweite Phosgenzersetzungseinrichtung (3012), wobei die erste und die zweite Phosgenzersetzungseinrichtung getrennt voneinander mit Phosgen-haltigen Abgasströmen anströmbar sind;
D) eine Verbrennungseinrichtung (6000) geeignet zur Verbrennung des in Abgasaufarbeitungsstrecke (3000) anfallenden aufgearbeiteten Abgases;
wobei während der Herstellung des chemischen Produkts (1) in der Reaktionsstrecke (1000) Phosgen (3) im stöchiometrischen Überschuss bezogen auf alle aktiven Wasserstoffatome des H-funktionellen Reaktanten eingesetzt wird, wobei der in der Reaktionszone (1200) aus A.II) erhaltene Phosgen-haltige Prozessabgasstrom (70) und der in der Trenneinheit (2100-2500) aus B.I) anfallende Phosgen-haltige Prozessabgasstrom (170), jeweils optional nach Durchlaufen weiterer Aufarbeitungsschritte, der ersten Phosgenzersetzungseinrichtung (3011) zugeführt werden,
wobei ferner die Produktion des chemischen Produkts (1) zeitweise durch Abstellen der Zufuhr des H-funktionellen Reaktanten (2) unterbrochen wird,
wobei während dieser Produktionsunterbrechung
- mindestens ein Anlagenteil aus A) und/oder B) außer Betrieb genommen und in mindestens einem der nicht außer Betrieb genommenen Anlagenteile ein Ausgangsstrom dieses mindestens einen nicht außer Betrieb genommenen Anlagenteils
   (i) in den jeweiligen Anlagenteil oder
   (ii) in einen strömungstechnisch davor oder dahinter liegenden Anlagenteil geführt und von dort, optional über weitere nicht außer Betrieb genommene Anlagenteile, in den ausgehenden Anlagenteil
   zurückgeführt und so der jeweilige Anlagenteil in Kreislauffahrweise gebracht wird, wobei in dem mindestens einen in Kreislauffahrweise gebrachten Anlagenteil Prozessabgas und in dem mindestens einen außer Betrieb genommenen Anlagenteil Sauerstoff-haltiges Abgas (400-X) anfällt;
- Prozessabgas aus dem mindestens einen in Kreislauffahrweise gebrachten Anlagenteil in die erste Phosgenzersetzungseinrichtung (3011) geführt wird, wobei die erste Phosgenzersetzungseinrichtung (3011) auch während der Produktionsunterbrechung in Betrieb bleibt;
- Sauerstoff-haltiges Abgas (400-X) aus dem mindestens einen außer Betrieb genommenen Anlagenteil der zweiten Phosgenzersetzungseinrichtung (3012) zugeführt wird, wobei die zweite Phosgenzersetzungseinrichtung (3012) mindestens während der Produktionsunterbrechung in Betrieb ist, wobei
- von Phosgen befreites Prozessabgas (210-X) aus der ersten Phosgenzersetzungseinrichtung (3011) und von Phosgen befreites Sauerstoff-haltiges Abgas (410-X) aus der zweiten Phosgenzersetzungseinrichtung (3012) getrennt voneinander der Verbrennungseinrichtung (6000) an räumlich getrennten Stellen zugeführt und dort verbrannt werden.

Die Erfindung betrifft ferner eine **Produktionsanlage zur Herstellung eines chemischen Produkts** (1) durch Umsetzung eines H-funktionellen Reaktanten (2) mit Phosgen (3), aufweisend folgende Anlagenteile:
A) eine Reaktionsstrecke (1000) geeignet zur Umsetzung eines H-funktionellen Reaktanten (2) mit Phosgen (3), aufweisend:
   A.I) eine Mischzone (1100) zur Vermischung des H-funktionellen Reaktanten (2) und Phosgen (3) zu einem Reaktionsgemisch (50),
   A.II) eine mit der Mischzone (1100) verbundene Reaktionszone (1200) zur Umsetzung des in A.I) erhaltenen Reaktionsgemisches (50) unter Ausbildung einer Flüssigphase (60) enthaltend das chemische Produkt (1) sowie Phosgen (3) und eines Phosgen-haltigen Prozessabgasstroms (70);
B) eine mit der Reaktionsstrecke (1000) verbundene Aufarbeitungsstrecke (2000) aufweisend:
   B.I) eine Trenneinheit (2100-2500) zur Trennung der in A.II) erhaltenen Flüssigphase (60) in einen Phosgen-haltigen Prozessabgasstrom (170) und in eine Flüssigphase (100) enthaltend das chemische Produkt (1);
C) eine Abgasaufarbeitungsstrecke (3000) geeignet zur Aufarbeitung während der Herstellung des chemischen Produkts (1) und während der Produktionsunterbrechung anfallender Phosgen-haltiger Abgasströme, aufweisend eine erste Phosgenzersetzungseinrichtung (3011) und eine zweite Phosgenzersetzungseinrichtung (3012), wobei die erste und die zweite Phosgenzersetzungseinrichtung getrennt voneinander mit Phosgen-haltigen Abgasströmen anströmbar sind;
D) eine Verbrennungseinrichtung (6000) geeignet zur Verbrennung des in Abgasaufarbeitungsstrecke (3000) anfallenden aufgearbeiteten Abgases, wobei die Verbrennungseinrichtung (6000) so mit der Abgasaufarbeitungsstrecke (3000) verbunden ist, dass die in der ersten Phosgenzersetzungseinrichtung (3011) und in der zweiten Phosgenzersetzungseinrichtung (3012) anfallenden Abgasströme der Verbrennungseinrichtung (6000) getrennt voneinander zugeführt werden;
wobei die Produktionsanlage derart ausgestaltet ist, dass bei einer Produktionsunterbrechung durch Abstellen der Zufuhr des H-funktionellen Reaktanten (2) und Außerbetriebnahme mindestens eines Anlagenteils ein Ausgangsstrom dieses mindestens einen nicht außer Betrieb genommenen Anlagenteils
(i) in den jeweiligen Anlagenteil oder
(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt und so der jeweilige Anlagenteil in Kreislauffahrweise gebracht werden kann,
wobei die Produktionsanlage ferner derart ausgestaltet ist, dass
- in dem mindestens einen in Kreislauffahrweise gebrachten Anlagenteil anfallendes Prozessabgas in die erste Phosgenzersetzungseinrichtung (3100) und
- in dem mindestens einen außer Betrieb genommenen Anlagenteil anfallendes Sauerstoff-haltiges Abgas (400-X) in die zweite Phosgenzersetzungseinrichtung (3200)
geführt werden kann, ohne dass Prozessabgas und Sauerstoff-haltiges Abgas (400-X) miteinander vermischt werden.

*"H funktionelle Reaktanten (2)"* im Sinne der Erfindung sind solche organischen Verbindungen, die mindestens ein an N, O, oder S gebundenes Wasserstoffatom aufweisen, das mit Phosgen unter Freisetzung von Chlorwasserstoff zu reagieren vermag (im Rahmen der Erfindung als *"aktives Wasserstoffatom* " bezeichnet). *"Chemische Produkte (1)"* im Sinne der Erfindung sind die dabei gebildeten organischen Kondensationsprodukte. Unter der Funktionalität wird die Anzahl an aktiven Wasserstoffatomen pro Molekül des H-funktionellen Reaktanten verstanden. In bestimmten Ausführungsformen können H-funktionelle Reaktanten auch in Form ihrer Salze eingesetzt werden; dies gilt erfindungsgemäß als von dem Begriff *"H-funktioneller Reaktant"* umfasst.

Das erfindungsgemäße Verfahren wird im Regelbetrieb kontinuierlich betrieben. Dies bedeutet, dass die Anlagenteile aus A) und B) während der Produktion, also während die Zufuhr des H-funktionellen Reaktanten nicht unterbrochen ist (und natürlich auch die Zufuhr von Phosgen nicht unterbrochen ist), kontinuierlich mit den entsprechenden Eingangsströmen beschickt werden (z. B. die Mischzone 1100 mit dem H-funktionellen Reaktanten und Phosgen) und ihnen kontinuierlich die jeweiligen Produkte entnommen werden (z. B. das Reaktionsgemisch 50). Entsprechendes gilt natürlich auch für die Abgasaufarbeitungsstrecke (3000) aus C) und die Verbrennungseinrichtung (6000) aus D).

Das erfindungsgemäße Merkmal, dass "*der in der Reaktionszone (1200) aus A.II) erhaltene Phosgen-haltige Prozessabgasstrom (70) und der in der Trenneinheit (2100-2500) aus B.I) anfallende Phosgen-haltige Prozessabgasstrom (170), jeweils optional nach Durchlaufen weiterer Aufarbeitungsschritte, der ersten Phosgenzersetzungseinrichtung (3011) zugeführt werden"* umfasst auch solche Ausführungsformen, in denen einer der beiden Prozessabgasströme in dem anderen aufgeht. Beispiele hierfür werden weiter unten beschrieben.

Im Rahmen der vorliegenden Erfindung wird in allen Ausführungsformen *"ein Anlagenteil aus A) und*/*oder B) außer Betrieb genommen".* Ein solches Außerbetriebnehmen eines Anlagenteils meint dabei dessen Stilllegung so, dass in dem Anlagenteil eine Revisions-, Reparatur-, Wartungs- oder Reinigungsmaßnahme (fortan summarisch Maßnahme) durchgeführt werden kann. Zu diesem Zweck wird die Zufuhr der im Regelbetrieb in diesen Anlagenteil geleiteten Reaktanten unterbrochen, und die Anbindung des betreffenden Anlagenteils an die übrigen Anlagenteile der Produktionsanlage wird abgesperrt, gegebenenfalls mit Ausnahme von Leitungen, die während der Maßnahme benötigt werden, beispielsweise zum Durchspülen des Anlagenteils mit einer Reinigungsflüssigkeit.

Der Begriff *"Außerbetriebnahme"* umfasst demnach erfindungsgemäß bei Vorhandensein von n Anlagenteilen im Sinne der vorliegenden Erfindung (siehe hierzu auch den folgenden Absatz), wobei n eine natürliche Zahl ist, die Außerbetriebnahme von maximal n - 1 dieser Anlagenteile. Erfindungsgemäß wird also mindestens ein Anlagenteil nicht im zuvor genannten Sinne *"außer Betrieb genommen"* (d. h. mindestens ein Anlagenteil wird nicht vollständig stillgelegt). Bevorzugt befasst sich die vorliegende Erfindung mit dem Fall der Außerbetriebnahme von 1 bis 2 Anlagenteilen, besonders bevorzugt von 1 Anlagenteil. Erfindungsgemäß wird daher bei Außerbetriebnahme eines Anlagenteils (oder mehrerer Anlagenteile, jedoch nicht aller Anlagenteile) in jedem Fall die Bildung weiteren Produkts unterbrochen (da die Zufuhr des H-funktionellen Reaktanten unterbrochen wird und daher kein weiteres Produkt mehr produziert werden kann).

Unter *"Kreislauffahrweise"* wird im Rahmen dieser Erfindung verstanden, dass ein Ausgangstrom eines Anlagenteils aus A) und/oder B) letztlich wieder als Eingangsstrom dieses Anlagenteils verwendet wird. Infolge der Unterbrechung der Produktion haben diese Ströme in der Kreislauffahrweise eine andere Zusammensetzung als im Regelbetrieb, was im Rahmen der vorliegenden Erfindung durch ein dem jeweiligen Bezugszeichen nachgestelltes "-X" angezeigt wird. Diese Kreislauffahrweise kann so umgesetzt werden, dass der Ausgangsstrom unmittelbar wieder in denselben Anlagenteil zurückgeführt wird. Es ist auch möglich (und bevorzugt), dass der Ausgangsstrom des ausgehenden Anlagenteils erst nach Durchlaufen eines weiteren Anlagenteils oder mehrerer weiterer Anlagenteile in den ausgehenden Anlagenteil zurückgeführt wird, wobei der weitere Anlagenteil oder die weiteren Anlagenteile dem ausgehenden Anlagenteil strömungstechnisch vor- oder nachgeschaltet sein können. Dabei kann der Begriff "*ein Anlagenteil aus A)"* sowohl für die Reaktionsstrecke (1000) als Ganzes stehen (also A.I) und A.II) beinhalten) als auch für jeden Bestandteil A.I) und A.II) für sich genommen stehen. Entsprechendes gilt für die Aufarbeitungsstrecke (2000) (die in bevorzugten Ausführungsformen weitere Bestandteile B.II) und B.III) aufweisen kann). In bestimmten Ausführungsformen können Anlagenteile auch aus einzelnen Apparaten aufgebaut sein, die dann ihrerseits Anlagenteile im Sinne der Erfindung sein können. In einem solchen Fall muss sich die Kreislauffahrweise nicht notwendigerweise über den (Gesamt-)Anlagenteil erstrecken, sondern kann auf einzelne Apparate desselben, d. h. (Unter-)Anlagenteile, beschränkt sein. Ein Beispiel hierfür ist die *"Trenneinheit (2100-2500)"* aus B.I), die aus verschiedenen Untereinheiten (2100, 2200, 2300 und 2500) aufgebaut sein kann, von denen wiederum jede als Anlagenteil im Sinne der Erfindung aufgefasst werden kann, wie weiter unten noch näher erläutert wird. Ein anderes Beispiel für einen aus mehreren Apparaten bestehenden Anlagenteil ist, in bestimmten Ausführungsformen, die Reaktionsstrecke (1000), denn Mischzone (1100) und Reaktionszone (1200) können auch Bestandteil ein und desselben Apparats (ein und desselben Anlagenteils) sein, wobei dann eine strikte räumlich Abgrenzung zwischen beiden unter Umständen nicht mehr möglich ist. Eine erfindungsgemäße Kreislauffahrweise kann also bspw. die gesamte Reaktionsstrecke (1000) (insbesondere, aber nicht darauf beschränkt, wenn Mischzone (1100) und Reaktionszone (1200) in einem Apparat realisiert sind) oder auch nur Teile davon umfassen. Es ist ebenfalls möglich, dass Anlagenteile aus A) mit Anlagenteilen aus B) von einer gemeinsamen Kreislauffahrweise umfasst sind.

Abgasströme, die im Regelbetrieb der Anlage oder bei der zuvor erläuterten Kreislauffahrweise anfallen, werden im Rahmen dieser Erfindung als *"Prozessabgasströme"* oder kurz als *"Prozessabgas"* bezeichnet. Solche Prozessabgasströme enthalten Sauerstoff regelmäßig allenfalls in einem untergeordneten Volumenanteil, der in jedem Fall (deutlich) kleiner ist als derjenige der im Folgenden beschriebenen Sauerstoff-haltigen Abgasströme. Dieser allenfalls untergeordnete Sauerstoffanteil kann etwa aus einem geringfügigen Sauerstoffanteil, der in den eingesetzten Reaktanten gelöst ist, oder, bei unter vermindertem Druck betriebenen Anlagenteilen, aus dem Vakuumsystem stammen. Vorzugsweise beträgt der auf das Gesamtvolumen bezogene Volumenanteil an Sauerstoff in solchen Prozessabgasströmen maximal 3,9 %, besonders bevorzugt maximal 3,0 %, ganz besonders bevorzugt maximal 2,6 %, außerordentlich ganz besonders bevorzugt maximal 2,0 %.

Abgasströme, die nur bei einer Maßnahme in einem außer Betrieb genommenen Anlagenteil anfallen, beispielsweise infolge einer aus Arbeitssicherheitsgründen betriebenen Absaugung, enthalten dagegen regelmäßig beträchtliche Anteile an Sauerstoff und werden daher im Rahmen der vorliegenden Erfindung als *"Sauerstoff-haltige Abgasströme"* oder kurz als *"Sauerstoff-haltiges Abgas"* (oder im betrieblichen Jargon als "Abluft") bezeichnet. Sauerstoff-haltig in diesem Sinne ist ein Abgasstrom, wenn sein auf das Gesamtvolumen bezogener Volumenanteil an Sauerstoff mindestens 4,0 % beträgt.

Dem Fachmann sind geeignete Methoden zur Bestimmung des Sauerstoffgehalts in Abgasströmen bekannt. Grundsätzlich liefern gängige Sauerstoffbestimmungsmethoden im Rahmen für die Zwecke der vorliegenden Erfindung insignifikanter Schwankungen das gleiche Ergebnis. Im Zweifelsfall ist das mit einem auf der Basis des paramagnetischen Wechseldruckverfahrens arbeitenden Sauerstoffsensor gemessene Ergebnis für die Zwecke der vorliegenden Erfindung maßgeblich. Geeignete Messapparate können beispielsweise von der Firma Siemens bezogen werden (z. B. "Oximat 6").

Erfindungsgemäß werden Prozessabgasströme und Sauerstoff-haltige Abgasströme vom Ort ihrer Entstehung bis zur Einleitung in die Verbrennungseinrichtung (6000) *getrennt gehalten.* Dies wird ermöglicht durch den Einsatz von mindestens zwei Phosgenzersetzungseinrichtungen (3011, 3012, ....), von denen eine (3011) nur für Prozessabgas und eine (3012) nur für Sauerstoff-haltiges Abgas vorgesehen ist (d. h. Prozessabgas und Sauerstoff-haltiges Abgas werden nie in dieselbe Phosgenzersetzungseinrichtung eingeleitet). Von der ersten Phosgenzersetzungseinrichtung (3011) für Prozessabgas führen eigene Abgasleitungen in die Verbrennungseinrichtung (6000), genauso wie von der zweiten Phosgenzersetzungseinrichtung (3012) für Sauerstoff-haltiges Abgas eigene Abgasleitungen in die Verbrennungseinrichtung (6000) führen. Diese *voneinander getrennten* Abgasleitungen aus der ersten Phosgenzersetzungseinrichtung (3011) und aus der zweiten Phosgenzersetzungseinrichtung (3012) ermöglichen es, Prozessabgas und Sauerstoff-reiches Abgas *ohne Vorvermischung* in die Verbrennung zu führen. Dabei münden die Abgasleitungen für Prozessabgas und für Sauerstoff-haltiges Abgas *"an räumlich getrennten Stellen"* in die Verbrennungseinrichtung (6000), sodass ein Vermischen von Bestandteilen aus beiden Strömen erst einsetzen kann, *wenn die Verbrennung bereits eingesetzt hat.* Die vorliegende Erfindung ermöglicht es somit, Prozessabgas und Sauererstoff-haltiges Abgas vom Ort der jeweiligen Entstehung bis zur Verbrennung voneinander getrennt zu halten, wodurch eine Explosionsgefahr ohne den zusätzlichen Einsatz eines inerten Verdünnungsgases wie beispielsweise Stickstoff vermieden werden kann.

Es folgt zunächst eine Kurzzusammenfassung verschiedener möglicher **Ausführungsformen der Erfindung:**
In einer **ersten Ausführungsform** der Erfindung ist das chemische Produkt (1) ein organisches Carbonat, insbesondere ein Polycarbonat ist.

In einer **zweiten Ausführungsform** der Erfindung ist das chemische Produkt (1) ein Isocyanat, insbesondere Toluylendiisocaynat oder ein Gemisch aus Methylendiphenylendiisocyanat und Polymethylenpolyphenylenpolyisocanat.

In einer **dritten Ausführungsform** der Erfindung, die eine besondere Ausgestaltung der zweiten Ausführungsform ist, weist die Trenneinheit (2100-2500) aus B.I) Folgendes auf:
- eine Destillationsvorrichtung (2100) zur Trennung des flüssigen Stroms (60) in einen flüssigen, Lösungsmittel und Isocyanat enthaltenden Strom (80) und einen gasförmigen, Phosgen und Chlorwasserstoff enthaltenden Prozessabgasstrom (90);
- eine Destillationsvorrichtung (2200) zur Trennung des flüssigen Lösungsmittel und Isocyanat enthaltenden Stroms (80) in einen Lösungsmittel enthaltenden Prozessabgasstrom (110) und eine flüssigen, Isocyanat enthaltenden Strom (100);
- eine Destillationsvorrichtung (2300) zur Trennung des Lösungsmittel enthaltenden Prozessabgasstroms (110) in einen flüssigen, Lösungsmittel enthaltenden Strom (120) und einen gasförmigen, Phosgen-haltigen Prozessabgasstrom (130).

In einer **vierten Ausführungsform** der Erfindung, die eine besondere Ausgestaltung der dritten Ausführungsform ist, weist die Trenneinheit (2100-2500) aus B.I) weiterhin eine Absorptionsvorrichtung (2500) auf, in welcher die Phosgen-haltigen Prozessabgasströme (70), (90) und (130) durch Absorption in einem Lösungsmittel (4) unter Erhalt eines flüssigen, Lösungsmittel und Phosgen enthaltenden Stroms (160) und eines gasförmigen, Chlorwasserstoff und Lösungsmittel enthaltenden Prozessabgasstroms (170) gereinigt werden, wobei bevorzugt die gasförmigen Phosgen-haltigen Prozessabgasströme (70) und (90) zunächst vereint werden und der vereinigte Phosgen-haltige Prozessabgasstrom aus (70) und (90) sowie der Phosgen-haltige Prozessabgasstrom (130) jeweils kondensiert und dann flüssig in die Absorptionsvorrichtung (2500) eingetragen werden.

In einer **fünften Ausführungsform** der Erfindung, die eine besondere Ausgestaltung der zweiten, dritten und vierten Ausführungsform, insbesondere der vierten Ausführungsform, ist, weist die Aufarbeitungsstrecke (2000) aus B) zusätzlich zur Trenneinheit (2100-2500) aus B.I) Folgendes auf:
B.II) eine Abtrenneinheit (2600) zur Abtrennung von Chlorwasserstoff aus dem Phosgen-haltigen Prozessabgasstrom (170),
in welcher der Phosgen-haltige Prozessabgasstrom (170) an Chlorwasserstoff abgereichert wird, wobei, bevorzugt nach Durchlaufen eines Brüdenkondensators (2630), ein gasförmiger, Lösungsmittel und ggf. gasförmige Nebenkomponenten enthaltender Phosgen-haltiger Prozessabgasstrom (200) erhalten wird, wobei der Phosgen-haltige Prozessabgasstrom (200) der ersten Phosgenzersetzungseinrichtung (3011) zugeführt wird.

In einer **sechsten Ausführungsform** der Erfindung, die eine besondere Ausgestaltung der fünften Ausführungsform ist, wird die Abtrennung des Chlorwasserstoffs in der Abtrenneinheit (2600) durch Absorption von Chlorwasserstoff in Wasser oder Salzsäure einer Konzentration im Bereich von 0,50 Massen-% bis 15,0 Massen-%, bezogen auf die Gesamtmasse der Salzsäure, durchgeführt, wobei neben dem Lösungsmittel und ggf. gasförmige Nebenkomponenten enthaltenden Phosgen-haltigen Prozessabgasstrom (200) ein Salzsäure enthaltender Strom (190) erhalten wird.

In einer **siebten Ausführungsform** der Erfindung, die eine besondere Ausgestaltung der zweiten, dritten, vierten, fünften und sechsten Ausführungsform ist, weist die Aufarbeitungsstrecke (2000) aus B) zusätzlich zur Trenneinheit (2100-2500) aus B.I) und, sofern vorhanden, zusätzlich zur Abtrenneinheit (2600) aus B.II), Folgendes auf:
B.III) eine Destillationseinheit (2400) zur Aufarbeitung der Flüssigphase (100) enthaltend das Isoycanat,
wobei der Destillationseinheit (2400) optional eine Einrichtung zur Abtrennung polymerer Isocyanatfraktionen (2410) vorgeschaltet ist.

In einer **achten Ausführungsform** der Erfindung, die eine besondere Ausgestaltung der siebten Ausführungsform ist, ist die Einrichtung zur Abtrennung polymerer Isocyanatfraktionen (2410) vorhanden, und während einer Produktionsunterbrechung werden folgende Kreislauffahrweisen etabliert:
- eine erste Kreislauffahrweise ausgehend vom Kopf der Destillationsvorrichtung (2200) über die Destillationsvorrichtung (2300) zurück in die Destillationsvorrichtung (2200);
- eine zweite Kreislauffahrweise ausgehend vom Sumpf der Destillationsvorrichtung (2200) über die Einrichtung zur Abtrennung polymerer Isocyanat-Fraktionen (2410) zurück in die Destillationsvorrichtung (2200);
- eine dritte Kreislauffahrweise ausgehend von der Destillationsvorrichtung (2100) zurück in dieselbe, und
- eine vierte Kreislauffahrweise ausgehend von der Abtrenneinheit (2600) zur Abtrennung von Chlorwasserstoff zurück in dieselbe,
wobei die Reaktionsstrecke (1000) aus A) außer Betrieb genommen wird.

In einer **neunten Ausführungsform** der Erfindung, die mit allen anderen Ausführungsformen kombiniert werden kann, verfügt die Produktionsanlage über eine Phosgenherstellungsstrecke (0) umfassend eine Vorrichtung (4000) zur Phosgenherstellung aus Kohlenstoffmonoxid (300) und Chlor (310), wobei die Herstellung von Phosgen bei der Produktionsunterbrechung, gegebenenfalls zeitversetzt nach Abstellung der Zufuhr des H-funktionellen Reaktanten (2), abgeschaltet wird.

In einer **zehnten Ausführungsform** der Erfindung, die mit allen anderen Ausführungsformen kombiniert werden kann, werden die Anlagenteile der Aufarbeitungsstrecke (2000) aus B) mindestens teilweise bei gegenüber Umgebungsdruck vermindertem Druck betrieben, wobei der verminderte Druck durch Vakuumerzeugungsanlagen erzeugt wird, in denen Prozessabgasströme anfallen, welche der ersten Phosgenzersetzungseinrichtung (3011) zugeführt werden.

In einer **elften Ausführungsform** der Erfindung, die eine besondere Ausgestaltung der zehnten Ausführungsform ist, weist die erste Phosgenzersetzungseinrichtung (3011) mindestens zwei getrennt voneinander betriebene Phosgenzersetzungs-Teilanlagen (3011-1, 3011-2) auf, wobei während der Herstellung des chemischen Produkts (1) eine dieser beiden Phosgenzersetzungs-Teilanlagen (3011-1) nur die Prozessabgasströme aus den Vakuumerzeugungsanlagen zugeführt werden, während der anderen Phosgenzersetzungs-Teilanlage (3011-2) alle übrigen Prozessabgasströme zugeführt werden, wobei während einer Produktionsunterbrechung die Vakuumerzeugungsanlagen außer Betrieb genommen werden und mit der Phosgenzersetzungs-Teilanlage (3011-1) verbunden bleiben.

In einer **zwölften Ausführungsform** der Erfindung, die mit allen anderen Ausführungsformen kombiniert werden kann, fallen während der Produktion des chemischen Produkts Sauerstoff-haltige Abgasströme (410-X) an, wobei die zweite Phosgenzersetzungseinrichtung (3012) für Sauerstoff-haltige Abgasströme auch während der Produktion des chemischen Produkts (1) betrieben wird und ihr die Sauerstoff-haltigen Abgasströme (410-X) zugeführt werden.

In einer **dreizehnten Ausführungsform** der Erfindung, die eine besondere Ausgestaltung der zwölften Ausführungsform ist, umfasst die zweite Phosgenzersetzungseinrichtung (3012) mindestens zwei parallel geschaltete Phosgenzersetzungs-Teilanlagen (3012-1, 3012-2), wobei einer der Phosgenzersetzungs-Teilanlagen (3012-1) nur die während der Produktion des chemischen Produkts anfallenden Sauerstoff-haltigen Abgasströme (410-X) zugeführt werden, während der anderen Phosgenzersetzungs-Teilanlage (3012-2) die bei einer Produktionsunterbrechung in dem mindestens einen außer Betrieb genommenen Anlagenteil anfallenden Sauerstoff-reichen Abgasströme (400-X) zugeführt werden.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

Das erfindungsgemäße Verfahren ist grundsätzlich zur Herstellung beliebiger **chemischer Produkte** (1) geeignet, die durch eine Kondensationsreaktion eines H-funktionellen Reaktanten mit Phosgen unter Abspaltung von Chlorwasserstoff gebildet werden. Der Verbleib des bei der Kondensationsreaktion gebildeten Chlorwasserstoffs hängt von der Verfahrensweise ab:
i. Wird der Chlorwasserstoff nicht neutralisiert und findet die Reaktion in Abwesenheit eines wässrigen Mediums statt, so geht Chlorwasserstoff in beträchtlichen Anteilen in den *Phosgen-haltigen Prozessabgasstrom* (70) über (neben gegebenenfalls weiteren Anteilen, die gelöst in der Flüssigphase (60) vorliegen können). In diesem Fall umfasst die Aufarbeitungsstrecke (2000) aus B) auch einen Anlagenteil B.II), eine Trenneinheit (2600) zur Abtrennung von Chlorwasserstoff aus dem in A.II) erhaltenen Phosgen-haltigen Prozessabgasstrom (70) (und aus der in B.I) erhaltenen Gasphase (170), deren Chlorwasserstoffgehalt aus in der Flüssigphase (60) gelöstem Chlorwasserstoff herrührt) unter Ausbildung eines *Phosgen-haltigen Prozessabgasstroms (200) abgereichert an Chlorwasserstoff* und eines *Chlorwasserstoff-haltigen (oder Salzsäure-haltigen) Stromes (190),* wie weiter unten noch näher erläutert werden wird.
ii. Findet die Reaktion (a) im Beisein eines wässrigen Mediums statt oder wird (b) die Flüssigphase (60) enthaltend das chemische Produkt unmittelbar nach Beendigung der Reaktion mit einem wässrigen Medium gewaschen, so wird eine wässrige Flüssigphase (61) erhalten, in die Chlorwasserstoff übergeht. Dies ist insbesondere dann der Fall, wenn das wässrige Medium eine Base enthält und so der Chlorwasserstoff in ein Salz überführt wird. Abhängig von der Vollständigkeit, mit der Chlorwasserstoff in die wässrige Flüssigphase (61) übergeht, ist ein Aufarbeitungsteil B.II) gegebenenfalls verzichtbar.

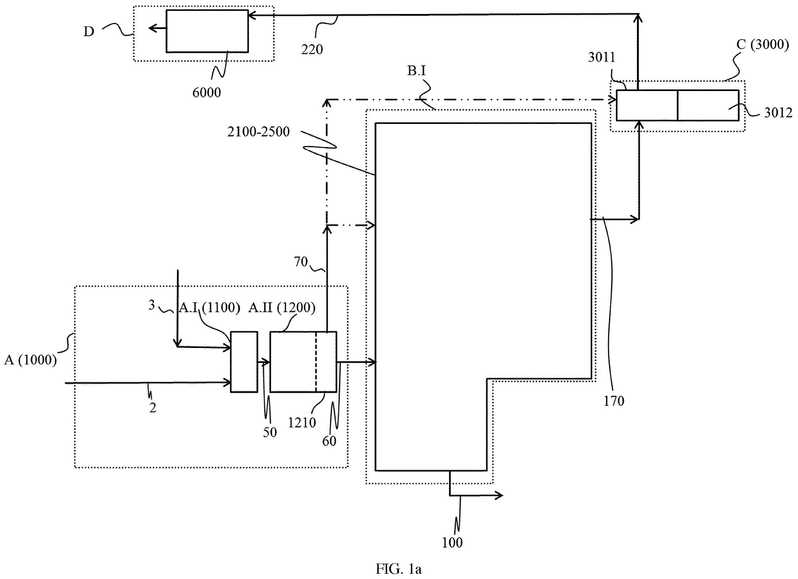

Die einfachste Ausführungsform des erfindungsgemäßen Verfahrens ohne Chlorwasserstoffabtrennung aus dem Prozessabgas im Regelbetrieb ist in **FIG. 1a** dargestellt:
Der in der Reaktionszone (1200) aus A.II) bzw. in einer in die Reaktionszone integrierten Abscheidezone (1210) anfallende Phosgen-haltige Prozessabgasstrom (70) wird entweder direkt oder nach Durchlaufen der Aufarbeitungsstufe B.I) in die erste Phosgenzersetzungseinrichtung (3011) geleitet. Wird die Aufarbeitungsstufe B.I) durchlaufen, so ist es zweckmäßig, den Phosgen-haltigen Prozessabgasstrom (70) in dieser Aufarbeitungsstufe mit dort anfallenden Prozessabgasströmen zu vereinen, sodass der Phosgen-haltige Prozessabgasstrom (70) letztlich im Phosgen-haltigen Prozessabgasstrom (170) *aufgeht.*

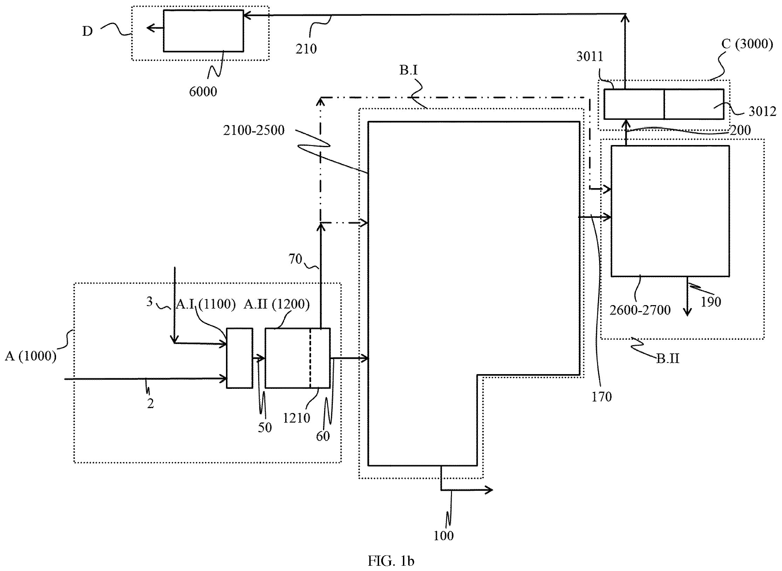

**FIG. 1b** zeigt eine Ausführungsform des erfindungsgemäßen Verfahrens mit Chlorwasserstoffabtrennung aus dem Prozessabgas im Regelbetrieb:
Der in der Reaktionszone (1200) aus A.II) bzw. in einer in die Reaktionszone integrierten Abscheidezone (1210) anfallende Phosgen-haltige Prozessabgasstrom (70) wird entweder direkt oder nach Durchlaufen der Aufarbeitungsstufe B.I) in die Chlorwasserstoffabtrenneinheit (2600) aus B.II) geleitet. Wird die Aufarbeitungsstufe B.I) durchlaufen, so ist es zweckmäßig, den Phosgen-haltigen Prozessabgasstrom (70) in dieser Aufarbeitungsstufe mit dort anfallenden Prozessabgasströmen zu vereinen, sodass der Phosgen-haltige Prozessabgasstrom (70) letztlich im Phosgen-haltigen Prozessabgasstrom (170) *aufgeht.*

Bekannte Beispiele für auf diese Weise zugängliche chemische Produkte (1) sind *organische Carbonate,* insbesondere *Polycarbonate* (durch Umsetzung von organischen Alkoholen, insbesondere mehrwertigen Alkoholen, mit Phosgen) und *Isocyanate* (durch Umsetzung von organischen primären Aminen mit Phosgen). Die Grenzflächenpolykondensation von Alkoholsalzen (2) in basischer wässriger Lösung und Phosgen (3), das in einem organischen Lösungsmittel (4) gelöst ist, zur Herstellung von Polycarbonaten (1) ist ein Beispiel für die zuvor genannte Verfahrensführung ii in der Variante (a). Die Herstellung von Isocyanaten (1) durch Phosgenierung der korrespondierenden primären Amine (2) ist ein Beispiel für Verfahrensführung i. Die Erfindung wird im Folgenden beispielhaft anhand der Herstellung von organischen Carbonaten, insbesondere Polycarbonaten, sowie Isocyanaten erläutert; sie ist jedoch nicht hierauf beschränkt.

Das erfindungsgemäße Verfahren ist auf die Herstellungsverfahren beliebiger *organischer Carbonate* und insbesondere *Polycarbonate* anwendbar. Bevorzugt wird das erfindungsgemäße Verfahren eingesetzt in Verfahren zur Herstellung von Polycarbonaten, die sich von Bisphenol S, Dihydroxydiphenylsulfid, Tetramethylbisphenol A, 1,1-Bis(4-hydroxyphenyl)-3,3,5-trimethylcyclohexan (BPTMC), 1,1,1-Tris(4-hydroxyphenyl)-ethan (THPE) oder Bishenol A ableiten. Bisphenol A ist besonders bevorzugt.

Das erfindungsgemäße Verfahren ist auf die Herstellungsverfahren beliebiger aromatischer, aliphatischer und araliphatischer *Isocyanate* anwendbar. Bevorzugt eingesetzt wird das erfindungsgemäße Verfahren zur Herstellung von Methylendiphenylendiisocynat (aus Methylendiphenylendiamin), Polymethylenpolyphenylenpolyisocyanat (aus Polymethylenpolyphenylenpolyamin), Gemischen aus Methylendiphenylendiisocynat und Polymethylenpolyphenylenpolyisocyanat, Toluylendiisocyanat (aus Toluylendiamin), Xylylendiisocyanat (aus Xylylendiamin), 1,5-Pentandiisocyanat (aus 1,5-Pentandiamin), Hexamethylendiisocyanat (aus Hexamethylendiamin), Isophorondiisocyanat (aus Isophorondiamin) und Naphthyldiisocyanat (aus Naphthyldiamin), besonders bevorzugt von Methylendiphenylendiisocyanat, Gemischen aus Methylendiphenylendiisocyanat und Polymethylenpolyphenylenpolyisocyanat sowie Toluylendiisocyanat. Ganz besonders bevorzugt eignet sich das erfindungsgemäße Verfahren zur Herstellung von Methylendiphenylendiisocyanat und Gemischen aus Methylendiphenylendiisocyanat und Polymethylenpolyphenylenpolyisocyanat.

Die **Umsetzung des H-funktionellen Reaktanten mit Phosgen in der Reaktionsstrecke (1000) aus A) im Regelbetrieb** kann, abhängig vom umzusetzenden H-funktionellen Reaktanten, in der Gas- oder Flüssigphase erfolgen.

Bei Flüssigphasenreaktionen ist es üblich und auch im erfindungsgemäßen Verfahren vorgesehen, den H-funktionellen Reaktanten (2) und/oder Phosgen (3) in einem organischen Lösungsmittel (4) zu lösen, dann beide Reaktionspartner zu vermischen (A.I)) und anschließend zur Reaktion zu bringen (A.II)). Dabei können beide Reaktionspartner in einem organischen Lösungsmittel (4) gelöst sein oder auch nur einer, wobei dann der andere entweder in Substanz (ohne vorherige Auflösung) oder in wässriger Lösung eingesetzt wird.

Bei Gasphasenreaktionen ist es üblich und auch im erfindungsgemäßen Verfahren vorgesehen, das gasförmige Rohprodukt durch direkte (d. h. unter stofflichem Kontakt mit einem Kühlmedium) oder indirekte Abkühlung (d. h. ohne stofflichen Kontakt mit einem Kühlmedium) soweit zu verflüssigen, dass das gewünschte chemische Produkt (1) in der sich ausbildenden Flüssigphase erhalten wird (bis auf gegebenenfalls kleine Anteile, die zunächst in der Gasphase verbleiben und durch nachgelagerte Aufarbeitungsschritte zurückgewonnen werden). Als Kühlmedium eignet sich dabei insbesondere ein organisches Lösungsmittel (4), rezykliertes und abgekühltes chemisches Produkt (1) oder eine Mischung aus beiden. Auf diese Weise wird auch bei Gasphasenreaktionen eine Flüssigphase (60) enthaltend das chemische Produkt (1) erhalten.

Die Umsetzung von Alkoholen (2) mit Phosgen (3) zu (Poly-)Carbonaten (1) erfolgt in flüssiger Phase, bevorzugt als Grenzflächenpolykondensation. Dabei wird der Alkohol (2) in einer wässrigen Lösung einer Base (bevorzugt Alkalimetallhydroxid, insbesondere Natriumhydroxid), also in Form eines Alkoholats, mit in einem organischen Lösungsmittel (4) (bevorzugt ein aliphatischer Kohlenwasserstoff mit Halogensubstitution, insbesondere Mono, Di- oder Trichlormethan oder Tetrachlorethylen) gelösten Phosgen (3) zur Reaktion gebracht. Das gebildete (Poly-)Carbonat (1) geht in die organische Phase über, der freigesetzte Chlorwasserstoff geht in Form des entsprechenden Salzes in die wässrige Phase (61) über. Bei dieser Verfahrensführung sind Mischzone (1100) und Reaktionszone (1200) nicht streng voneinander zu trennen; der gesamte verwendete Misch- und Reaktionsapparat ist daher als ein Anlagenteil (1100-1200) aufzufassen. Überschüssiges Phosgen (3) bleibt teilweise in der Flüssigphase (60) gelöst und geht zum Teil in den Gasraum über dem flüssigen Reaktionsgemisch (*in den Phosgen-haltigen Prozessabgasstrom (70))* über. Die (organische) Flüssigphase (60) enthaltend das (Poly-)Carbonat (1) und die wässrige Flüssigphase (61) enthaltend den freigesetzten Chlorwasserstoff in Form des Salzes der eingesetzten Base werden voneinander getrennt. Mindestens die Flüssigphase (60) wird in B) aufgearbeitet.

Die Umsetzung von organischen primären Aminen mit Phosgen zu Isocyanaten erfolgt in einer Ausführungsform der Erfindung *in der Flüssigphase.* Dabei werden Amin (2) und Phosgen (3) in einem organischen Lösungsmittel (4) gelöst, die so erhaltenen Lösungen vermischt und zur Reaktion gebracht. Zur Vermischung in der Mischzone (1100) können sowohl (i) statische Mischorgane (vorzugsweise Düsen) als auch (ii) dynamische Mischorgane (vorzugsweise Rotor-Stator-Mischer) eingesetzt werden. Im ersteren Fall (i) sind Misch- und Reaktionszone bevorzugt in einem einzigen Apparat (einem einzigen Anlagenteil 1100-1200) angeordnet, beispielsweise indem ein Düsenrohr (gegebenenfalls auch mehrere Düsenrohre) zur Zufuhr eines der Ausgangsstoffe in einen dieses Düsenrohr (gegebenenfalls die mehreren Düsenrohre) umhüllenden Rohrreaktor, welchen der andere Ausgangsstoff durchströmt, hineinragt. Im letzteren Fall (ii) sind Misch- und Reaktionszone bevorzugt in zwei Apparaten angeordnet, in einem dynamischen Mischer (1100) mit nachgeschaltetem Verweilzeitreaktor (1200). Andere Ausgestaltungen des Falls (ii) (z. B. dynamische Mischeinrichtung und Verweilzeitzone *in einem Apparat*) sind jedoch nicht ausgeschlossen. In jedem Fall werden am Ende der kontinuierlich durchströmten Reaktionszone (1200) eine Flüssigphase (60) enthaltend das Isocyanat (1) (und Lösungsmittel (4) sowie Anteile gelösten Phosgens und Chlorwasserstoffs) und ein Phosgen-haltiger Prozessabgasstrom (70) (der in dieser Ausführungsform auch Chlorwasserstoff enthält) erhalten, die beide in B) aufgearbeitet werden.

Die Umsetzung von organischen primären Aminen mit Phosgen zu Isocyanaten erfolgt in einer anderen Ausführungsform der Erfindung *in der Gasphase.* Amin (2) und Phosgen (3) werden in die Gasphase überführt, vermischt und (immer noch im gasförmigen Zustand) zur Reaktion gebracht. Die Vermischung erfolgt hier vorzugsweise über Düsen wie zuvor für die Flüssigphasenreaktion beschrieben. Misch- und Reaktionszone sind dann in einem einzigen Mischer-Reaktor (1100-1200) angeordnet. Das dabei erhaltene rohe gasförmige Verfahrensprodukt wird durch Kontaktieren mit einer Quenchflüssigkeit (Lösungsmittel (4) und/oder rezykliertes Isocyanat) bei einer Temperatur unterhalb des Siedepunkts des Isocyanats (1) und oberhalb des Zersetzungspunktes des korrespondierenden Carbamoylchlorids rasch abgekühlt, wobei das gebildete Isocyanat in die sich ausbildende Flüssigphase übergeht. Auf diese Weise werden eine Flüssigphase (60) enthaltend das Isocyanat (1) (und die eingesetzte Quenchflüssigkeit sowie Anteile gelösten Phosgens und Chlorwasserstoffs) und ein Phosgen-haltiger Prozessabgasstrom (70) (der in dieser Ausführungsform auch Chlorwasserstoff enthält) erhalten, die beide in B) aufgearbeitet werden.

In allen Ausführungsformen der Erfindung, in denen ein organisches Lösungsmittel (4) eingesetzt wird, ist dieses bevorzugt ausgewählt aus der Gruppe bestehend aus aliphatischen Kohlenwasserstoffen ohne Halogensubstitution [vorzugsweise Decahydronaphthalin], aliphatischen Kohlenwasserstoffen mit Halogensubstitution [vorzugsweise Mono, Di- oder Trichlormethan oder Tetrachlorethylen], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho Dichlorbenzol] und Mischungen der vorgenannten organischen Lösungsmittel. Für die Herstellung von. Vorzugsweise werden Monochlorbenzol (MCB) oder ortho-Dichlorbenzol (ODB) eingesetzt.

Die **Aufarbeitung der in A.II) erhaltenen Flüssigphase (60) enthaltend das chemische Produkt (1) und der in A.II) erhaltenen Gasphase enthaltend Phosgen im Regelbetrieb** kann, abhängig vom umzusetzenden H-funktionellen Reaktanten und der Verfahrensweise in A), auf verschiedene Weisen realisiert werden, sie umfasst jedoch in jedem Fall die Abtrennung gasförmigen Phosgens aus der Flüssigphase (60) in der Trenneinheit (2100-2500). Geeignete Einrichtungen hierfür sind dem Fachmann geläufig, bevorzugt wird zu diesem Zweck eine Destillationskolonne oder eine Anordnung von mehreren Destillationskolonnen eingesetzt. Dabei wird eine Flüssigphase (100), die an Phosgen abgereichert ist und das chemische Produkt (1) enthält, neben einem das abgetrennte Phosgen enthaltenden Prozessabgasstrom (170) erhalten. Der so erhaltene Phosgen-haltige Prozessabgasstrom (170) wird, gegebenenfalls nach Durchlaufen weiterer Aufarbeitungsschritte, der ersten Phogenzersetzungseinrichtung (3011) zugeführt, deren Betriebsweise weiter unten näher erläutert wird.

Ein solcher gegebenenfalls durchgeführter weiterer Aufarbeitungsschritt umfasst insbesondere eine Abtrennung von Chlorwasserstoff aus dem Phosgen-haltigen Prozessabgasstrom (170) in dem weiter oben bereits erwähnten Anlagenteil B.II), der Trenneinheit (2600). Diese Ausführungsform wird dann durchgeführt, wenn die Flüssigphase (60) enthaltend das chemische Produkt (1) signifikante Anteile an Chlorwasserstoff enthält, was regelmäßig dann der Fall ist, wenn die Reaktion in A) in Abwesenheit eines wässrigen Mediums ohne Neutralisation des gebildeten Chlorwasserstoffs stattfindet, was insbesondere in der Herstellung von Isocyanaten der Fall ist. Dann enthält auch der in A.II) erhaltene Phosgen-haltige Prozessabgasstrom (70) in beträchtlichen Anteilen Chlorwasserstoff, der zweckmäßigerweise ebenfalls in der Trenneinheit (2600) abgetrennt wird. Geeignete Verfahren zur Abtrennung von Chlorwasserstoff aus gasförmigen Strömen sind dem Fachmann bekannt und können auch im Rahmen der vorliegenden Erfindung eingesetzt werden. Beispielhaft sei die Absorption des Chlorwasserstoffs in Wasser oder verdünnter Salzsäure (Salzsäure einer Konzentration im Bereich von 0,50 Massen-% bis 15,0 Massen-%) genannt. Unabhängig von der genauen Verfahrensweise fallen in B.II) ein Phosgen-haltiger Prozessabgasstrom (200), der an Chlorwasserstoff abgereichert ist, und ein Chlorwasserstoffhaltiger (bzw. Salzsäure-haltiger bei Absorption in wässrigem Medium) Strom (190) an.

In bevorzugten Ausführungsformen der Erfindung wird die Flüssigphase (100) abgereichert an Phosgen und enthaltend das chemische Produkt (1) in B.III), einer Reinigungseinheit (2400), aufgereinigt, um das herzustellende chemische Produkt (1) in möglichst reiner Form zu gewinnen. Die Reinigungseinheit (2400) umfasst bevorzugt eine Destillationskolonne oder eine Anordnung von mehreren in Reihe geschalteten Destillationskolonnen.

Die **Aufarbeitung des in B.I) bzw. B.II) erhaltenen Prozessabgasstroms (170) bzw. (200) in der Aufarbeitungsstrecke (3000) aus C) im Regelbetrieb** umfasst mindestens eine Stufe der Phosgenzersetzung. Erfindungsgemäß werden die Prozessabgasströme in die erste Phosgenzersetzungseinrichtung (3011) geführt. Diese wird dabei bevorzugt so betrieben, dass Phosgen katalytisch, bevorzugt an Aktivkohle, unter Verwendung eines wässrigen Stroms einer Temperatur im Bereich von 5,0 °C bis 50,0 °C, bevorzugt 20,0 °C bis 40,0 °C zersetzt wird. Der wässrige Strom kann Wasser, insbesondere Betriebswasser, Dampf-Kondensat (d. i. in Kondensatoren wie insbesondere Wärmeaustauschern anfallender kondensierter Heizdampf), vollentsalztes Wasser (VE-Wasser) oder eine Mischung mindestens zweier davon sein. Ebenfalls möglich ist der Einsatz von 1%iger bis 3%iger Salzsäure (Prozentangaben bezogen auf die Gesamtmasse der Salzsäure) als wässriger Strom. Hierzu bietet es sich an, den in der Phosgenzersetzungseinrichtung - infolge der Hydrolyse von Phosgen salzsauren - erhaltenen wässrigen Strom zu rezyklieren, wobei auch ein Teil des salzsauren wässrigen Stroms ausgeschleust werden kann, um eine zu hohe Aufkonzentrierung zu vermeiden. Der Einsatz von Dampf-Kondensat ist bevorzugt, insbesondere in Verbindung mit rezykliertem salzsaurem Strom aus der Phosgenzersetzungseinrichtung. Die Phosgenzersetzung erfolgt bevorzugt bei einem Druck im Bereich von 800 mbar bis 1000 mbar (absolut), bevorzugt 850 mbar bis 950 mbar (absolut), was vorzugsweise durch einen hinter der Phosgenzersetzungseinrichtung (3011) angebrachten Ventilator bewirkt wird. Die erste Phosgenzersetzungseinrichtung (3011) kann mehrere Teilanlagen umfassen, insbesondere mehrere, insbesondere zwei, in Reihe hintereinander geschaltete Rohrreaktoren, sog. "Phosgenvernichtungstürme". Dabei kann ein bevorzugt vorhandener Ventilator hinter jeder Teilanlage zur Phosgenzersetzung oder auch nur hinter der in Strömungsrichtung letzten Teilanlage zur Phosgenzersetzung angebracht sein.

Wenn das in die Aufarbeitungsstrecke (3000) geführte Prozessabgas noch substanzielle Anteile an Lösungsmittel (4) enthält, kann es zweckmäßig sein, dieses Lösungsmittel in einer der Phosgenzersetzung nachgeschalteten Lösungsmitteladsorption (in den Zeichnungen nicht dargestellt) an Aktivkohle zu adsorbieren und so abzutrennen.

Erfindungsgemäß umfasst die Aufarbeitungsstrecke (3000) aus C) mindestens eine erste Phosgenzersetzungseinrichtung (3011) für Prozessabgasströme und mindestens eine zweite Phosgenzersetzungseinrichtung (3012) für Sauerstoff-haltige Abgasströme. Die mindestens eine zweite Phosgenzersetzungseinrichtung (3012) für Sauerstoff-haltige Abgasströme ist bevorzugt so aufgebaut und wird bevorzugt so betrieben wie zuvor für die mindestens eine erste Phosgenzersetzungseinrichtung (3011) für Prozessabgas beschrieben. Die mindestens eine zweite Phosgenzersetzungseinrichtung (3012) wird im Regelbetrieb zwar nicht zwingend benötigt, es ist jedoch in vielen Fällen zweckmäßig, sie auch während des Regelbetriebs in Betrieb zu halten. Auf diese Weise wird es zum einen ermöglicht, Sauerstoff-haltige Abgasströme (410-X) aus Probeentnahmestellen über eigens dazu vorgesehene Einrichtungen zu entsorgen. Diese Einrichtungen zum Abführen Sauerstoff-haltiger Abgasströme aus Probeentnahmestellen können permanent angebrachte (fest verrohrte) Leitungen oder flexibel anbringbare Einrichtungen wie Schläuche aus Kunststoff (vorzugsweise Polyethylen), Gummi oder insbesondere Metall sein; in jedem Fall (egal ob fest verrohrt oder flexibel) ist es bevorzugt, die Einrichtungen zum Abführen Sauerstoff-haltiger Abgasströme aus einem elektrisch leitfähigem Material zu fertigen. Unabhängig davon ist es zum anderen grundsätzlich bevorzugt, fest verrohrte Leitungen von allen Phosgenführenden Anlagenteilen in die mindestens eine zweite Phosgenzersetzungseinrichtung (3012) vorzuhalten, durch welche im Falle einer Notabschaltung eines Anlagenteils bei einer Leckage Sauerstoff-haltiges Abgas der Phosgenzersetzung zugeführt werden kann, ohne dass hierfür erst in zeitraubender Weise die Voraussetzungen geschaffen werden müssen.

Die **Verbrennung des in C) erhaltenen aufgearbeiteten Prozessabgases (210) in der Verbrennungseinrichtung (6000) aus D)** kann grundsätzlich erfolgen wie aus dem Stand der Technik bekannt.

Aus Sicherheitsgründen ist es in allen Ausführungsformen der Erfindung bevorzugt, das für die Durchführung der Reaktion in A) erforderliche **Phosgen (3) vor Ort** durch Umsetzung von Kohlenstoffmonoxid (300) mit Chlor (310) in einer Phosgenherstellungsstrecke (0) umfassend eine Vorrichtung (4000) zur Phosgenherstellung **herzustellen.** Für die Ausführung dieses Schritts kann ein "Kaltvereiniger" entsprechend EP 1 640 341 B1 oder ein "Heißvereiniger" entsprechend EP 0 134 506 B1 benutzt werden. In jedem Verfahren entsteht ein Kohlenstoffmonoxid und Reste an Phosgen enthaltender Prozessabgasstrom (320), der vorzugsweise, insbesondere nach Durchlaufen weiterer Reinigungsschritte, der ersten Phosgenzersetzungseinrichtung (3011) zugeführt wird. In allen Ausführungsformen der Erfindung ist es bevorzugt, bei einer Produktionsunterbrechung die weitere Herstellung von Phosgen, gegebenenfalls zeitversetzt nach Abstellung der Zufuhr des H-funktionellen Reaktanten (2), abzustellen, um die Bildung großer Mengen Phosgens, die bis zur Wiederaufnahme der Produktion zwischengelagert werden müssten, zu vermeiden.

Im erfindungsgemäßen Verfahren können einzelne Anlagenteile im Regelbetrieb und bei Kreislauffahrweise bei einem gegenüber Umgebungsdruck vermindertem Druck betrieben werden. Dies betrifft insbesondere die Anlagenteile der Aufarbeitungsstrecke (2000) aus B). Die hierfür erforderlichen Vakuumerzeugungsanlagen produzieren Prozessabgasströme (in den Zeichnungen nicht gezeigt). Diese Prozessabgasströme werden (gegebenenfalls nach Durchlaufen weiterer Reinigungsschritte wie der Abtrennung von mitgerissenem Lösungsmittel (4); eine Chlorwasserstoffabtrennung wird im Allgemeinen nicht erforderlich sein und unterbleibt daher bevorzugt) durch Kondensation oder Tropfenabscheidung, ebenfalls der ersten Phosgenzersetzungseinrichtung (3011) zugeführt.

In einer bevorzugten Ausführungsform wird das Abgas des Vakuumsystems einer eigenen, nur für das Vakuumsystem bereitgestellten Phosgenzersetzungseinrichtung zugeführt. In einem solchen Fall besteht die erste Phosgenzersetzungseinrichtung (3011) aus mindestens zwei getrennt voneinander betriebenen Phosgenzersetzungs-Teilanlagen (3011-1, 3011-2). Diese Phosgenzersetzungs-Teilanlagen (3011-1, 3011-2) sind aufgebaut und werden betrieben wie zuvor für die erste Phosgenzersetzungseinrichtung (3011) beschrieben. Während der Herstellung des chemischen Produkts (1) im Regelbetrieb werden einer dieser beiden Phosgenzersetzungs-Teilanlagen (3011-1) *nur* die Prozessabgasströme aus den Vakuumerzeugungsanlagen zugeführt, während der anderen Phosgenzersetzungs-Teilanlage (3011-2) alle übrigen Prozessabgasströme zugeführt werden. Kommt es nun zu einer Leckage oder einem ähnlich schwerwiegenden Problem in den Vakuumerzeugungsanlagen, so werden diese außer Betrieb genommen, bleiben aber mit der ihnen zugeordneten Phosgenzersetzungs-Teilanlage (3011-1) verbunden, sodass sämtliche dort anfallende Abgasströme, seien sie nun Sauerstoff-haltig oder nicht, in eine eigene, von allen übrigen Abgasströmen getrennte, Phosgenzersetzung geführt werden. Die übrigen Anlagenteile der Produktionsanlage können dann in der beschriebenen Art und Weise in Kreislauffahrweise gebracht werden. In diesem Fall erfüllt die Phosgenzersetzungs-Teilanlage (3011-1) die gleiche Funktion wie in allen anderen Fällen die zweite Phosgenzersetzungseinrichtung (3012).

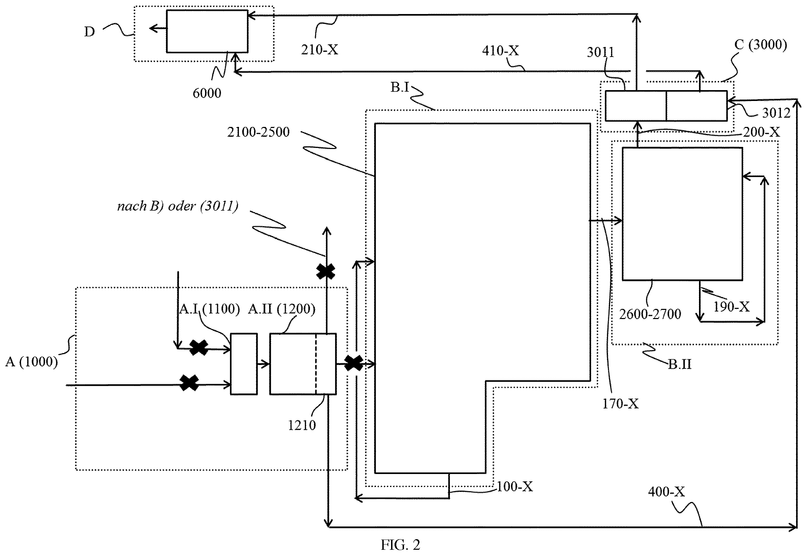

Die erfindungsgemäße Vorgehensweise bei einer **Produktionsunterbrechung** wird in **FIG. 2** am Beispiel der Außerbetriebnahme der gesamten Reaktionsstrecke (1000) aus A) dargestellt:
Die Leitungen für die Zu- und Abfuhr der Reaktanten und Produkte im Regelbetrieb sind geschlossen (dargestellt durch ein "**x**" in den entsprechenden Leitungen). Aus der Reaktionszone (1200) bzw. aus einer in die Reaktionszone integrierten Abscheidezone (1210) wird über eine dazu eigens vorgesehene Einrichtung Sauerstoff-haltiges Abgas (400-X) der zweiten Phosgenzersetzungseinrichtung (3012) zugeführt. Diese Einrichtung zum Abführen des Sauerstoff-haltigen Abgases (400-X) kann eine permanent angebrachte (fest verrohrte) Leitung sein. Es ist jedoch ebenfalls möglich, zu diesem Zweck eine flexibel anbringbare Einrichtung wie einen Schlauch aus Kunststoff (vorzugsweise Polyethylen), Gummi oder insbesondere Metall einzusetzen; in jedem Fall (egal ob fest verrohrt oder flexibel) ist es bevorzugt, die Einrichtungen zum Abführen Sauerstoff-haltigen Abgases aus einem elektrisch leitfähigem Material zu fertigen. Die Kreislauffahrweise wird in der Ausführungsform gemäß FIG. 2 realisiert, indem die flüssigen Ausgangsströme (100-X) aus B.I) und (190-X) aus B.II) in den jeweiligen Anlagenteil zurückgeführt werden (das Suffix "-X" zeigt an, dass diese Ströme eine andere Zusammensetzung haben als die entsprechenden Ströme des Regelbetriebs, (100) und (190)). Der Gasweg von B.I) nach B.II) ist offen (über Strom (170-X)). Ebenso ist der Gasweg von B.II) nach C) offen, das heißt Prozessabgas (200-X) gelangt in die erste Phosenzersetzungseinrichtung (3011). Der in der ersten Phosgenzersetzungseinrichtung (3011) erhaltene von Phosgen befreite Prozessabgasstrom (210-X) und der in der zweiten Phosgenzersetzungseinrichtung (3012) erhaltene von Phosgen befreite Prozessabgasstrom (410-X) werden über getrennte Leitungen räumlich verschiedenen Stellen der Verbrennungseinrichtung (6000) aus D) zugeführt und dort verbrannt.

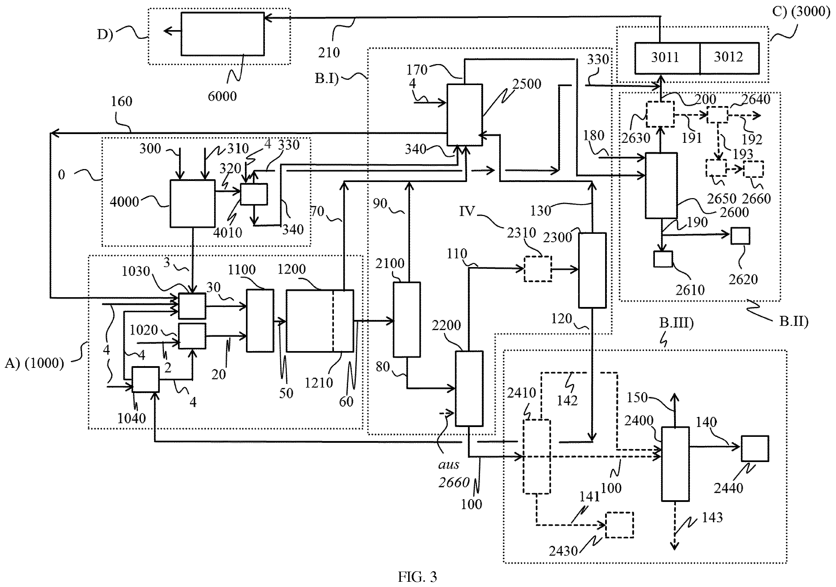

Zum besseren Verständnis der erfindungsgemäßen Vorgehensweise bei Produktionsunterbrechungen werden im Folgenden besonders bevorzugte Ausführungsformen des **Regelbetriebs der Herstellung von Isocyanaten** (1), die besonders bevorzugt chemische Produkte (1) im Sinne der Erfindung sind, im Detail näher beschrieben (vgl. hierzu **FIG. 3**).

Die kontinuierliche oder semi-kontinuierliche, bevorzugt kontinuierliche, **Produktion** des **Isocyanats in der Reaktionsstrecke (1000) aus A**) kann gemäß einem aus dem Stand der Technik bekannten Verfahren erfolgen. Geeignete Verfahren sind beispielsweise in EP 2 077 150 A1, EP 1 616 857 A1, EP 1 873 142 A1, EP 0 716 079 A1 und EP 0 314 985 B1 (Flüssigphasenverfahren) sowie EP 2 196 455 A1, EP 1 449 826 A1 und WO 2015/144681 A1 (Gasphasenverfahren) beschrieben. Konzentrationen und Mengenströme der Edukte Amin (2) und Phosgen (3) werden dabei jedoch bevorzugt so gewählt, dass sich in der Vermischung der Reaktionspartner in A.I) ein molares Verhältnis von Phosgen zu primären Aminogruppen von 1,1 : 1 bis 30 : 1, besonders bevorzugt von 1,25 : 1 bis 3 : 1, einstellt.

In einer bevorzugten Ausführungsform des erfindungsgemäßen Verfahrens werden Amin (2) und Phosgen (3) aus entsprechenden Vorratsbehältern (1020, 1030) der Vermischung in (1100) zugeführt. Dies geschieht bevorzugt in Form von Lösungen (20, 30) im Lösungsmittel (4). Bei Gasphasenprozessen kann auf die Lösung der Reaktanden in Lösungsmittel (4) verzichtet werden.

Phosgen (3) wird durch Umsetzung von Kohlenstoffmonoxid (300) mit Chlor (310) in der Vorrichtung (4000) - sog. "Phosgenvereiniger" - hergestellt. Dabei fällt ein Kohlenstoffmonoxid und Reste an Phosgen enthaltender Prozessabgasstrom (320) an, der vorzugsweise, insbesondere nach Durchlaufen weiterer Reinigungsschritte, der ersten Phosgenzersetzungseinrichtung (3011) zugeführt wird. Geeignete Reinigungsschritte umfassen dabei eine Absorption des Prozessabgasstroms (320) in Lösungsmittel (4) einer Temperatur im Bereich von 0,0 °C bis -20,0 °C in einer Absorptionskolonne (sog. "Phosgenwäscher"; (4010)), wobei ein Teil des Phosgens aus dem Kohlenstoffmonoxid und Reste an Phosgen enthaltenden Prozessabgasstrom (320) herausgewaschen werden. Der so erhaltene gewaschene Prozessabgasstrom (330) wird der ersten Phosgenzersetzungseinrichtung (3011) zugeführt, vorzugsweise wie in FIG. 3 gezeigt durch Vermischen mit Strom (200).

Geeignete Einrichtungen für die Ausgestaltung der Mischzone (1100) sind aus dem Stand der Technik hinreichend bekannt und wurden exemplarisch weiter oben bereits beschrieben. Nach der Vermischung wird das Reaktionsgemisch (50) in die Reaktionszone (1200) geleitet. Diese ist eine Verweilzeiteinrichtung, in welcher das in der Mischzone (1100) erhaltene Gemisch ausreichende Gelegenheit zum Ausreagieren erhält. Geeignete Apparate sind aus dem Stand der Technik hinlänglich bekannt. Die Trennung des rohen Verfahrensproduktes in die Flüssigphase (60) und den Phosgen-haltigen Prozessabgasstrom (70) erfolgt in der eigentlichen Reaktionszone selber oder in einer Abscheidezone (1210), die als Bestandteil von A.II) aufzufassen ist. Es ist auch möglich, die Mischzone und die Reaktionszone oder die Mischzone, die Reaktionszone und die Abscheidezone oder die Reaktionszone und die Abscheidezone in einen einzigen Apparat (z. B. in einen entsprechenden Reaktor) zu integrieren. Erfindungsgemäß können auch mehrere Mischzonen und/oder Reaktionszonen und/oder, sofern vorhanden, Abschcidczoncn, seriell oder parallel geschaltet sein; z. B. in Form einer Kaskade mehrerer in Serie geschalteter Reaktoren. Das in der Reaktionszone (1200) erhaltene Verfahrensprodukt trennt sich in eine Flüssigphase (60), enthaltend neben dem gewünschten Isoyanat gelösten Chlorwasserstoff, überschüssiges gelöstes Phosgen und Lösungsmittel, und einen gasförmigen Prozessabgasstrom (70), enthaltend weiterhin Chlorwasserstoffgas und gasförmiges Lösungsmittel. An die Reaktionszone kann sich erforderlichenfalls eine Vorrichtung zur Spaltung von Carbaminsäurechlorid (nicht gezeigt in FIG. 3) anschließen. Die Flüssigphase (60) durchläuft in einem solchen Fall diese Vorrichtung, bevor sie der Aufarbeitung in der Aufarbeitungsstrecke (2000) aus B) unterworfen wird. Die dabei entstehende an Chlorwasserstoff angereicherte Gasphase wird bevorzugt mit dem Phosgen-haltigen Prozessabgasstrom (70) vereint und gemeinsam weiter aufgearbeitet.

Diese **Aufarbeitung in Aufarbeitungsstrecke (2000) aus B**) umfasst zunächst einen Schritt der Abreicherung von Phosgen und Chlorwasserstoff aus Flüssigphase (60) aus A.I) durch Auftrennung dieses flüssigen Stroms (60) in einen flüssigen, Lösungsmittel und Isocyanat enthaltenden Strom (80) und einen gasförmigen, Phosgen und Chlorwasserstoff enthaltenden Prozessabgasstrom (90) in einer Destillationsvorrichtung (2100; sog. "Entphosgenierkolonne"). Diese sog. Entphosgenierkolonne kann nach einem beliebigen aus dem Stand der Technik bekannten Verfahren betrieben werden, bevorzugt wie in EP 1 854 783 B1, insbesondere in den Abschnitten [0018] und [0023] beschrieben.

Der so erhaltene flüssige Strom (80) wird in einer Destillationsvorrichtung (2200; sog. "Lösungsmittelkolonne") in einen noch Lösungsmittel enthaltenden Prozessabgasstrom (110) und eine flüssigen, Isocyanat enthaltenden Strom (100) aufgetrennt. Dies kann nach einem beliebigen aus dem Stand der Technik bekannten Verfahren erfolgen, bevorzugt wie in EP 1 854 783 B1, insbesondere in den Abschnitten [0024] bis [0027], beschrieben. Die Destillationsvorrichtung (2200) kann auch zwei oder mehr in Serie geschaltete Destillationskolonnen umfassen (in FIG. 3 wird aus Gründen der zeichnerischen Vereinfachung diese Möglichkeit nicht gezeigt).

Der Prozessabgasstrom (110) wird, bevorzugt nach Verflüssigung in einem Kondensator (2310), in einer Destillationsvorrichtung (2300; sog. "Lösungsmittelstripper") in einen flüssigen, Lösungsmittel enthaltenden Strom (120) und einen gasförmigen, Phosgen-haltigen Prozessabgasstrom (130) aufgetrennt. Dies kann nach einem beliebigen aus dem Stand der Technik bekannten Verfahren erfolgen, bevorzugt wie in EP 1 854 783 B1, insbesondere in den Abschnitten [0027] und [0028], beschrieben.

Die so erhaltenen Phosgen-haltigen Prozessabgasströme (70), (90) und (130) werden in einer Absorptionsvorrichtung (2500; sog. "Phosgenabsorber") durch Absorption in Lösungsmittel (4) unter Erhalt eines flüssigen, Lösungsmittel und Phosgen enthaltenden Stroms (160) und eines gasförmigen, Chlorwasserstoff und Lösungsmittel enthaltenden Prozessabgasstroms (170) gereinigt (d. h. von der Hauptmenge des Phosgens befreit), wobei bevorzugt die gasförmigen Phosgen-haltigen Prozessabgasströme (70) und (90) zunächst vereint werden und der vereinigte Phosgen-haltige Prozessabgasstrom aus (70) und (90) sowie der Phosgen-haltige Prozessabgasstrom (130) jeweils kondensiert und dann flüssig in die Absorptionsvorrichtung (2500) eingetragen werden. Diese Reinigung kann nach einem beliebigen aus dem Stand der Technik bekannten Verfahren erfolgen, bevorzugt wie in EP 2 093 215 A1 beschrieben. Der im Phosgenwäscher (4010) erhaltene mit Phosgen beladene Lösungsmittelstrom (340) wird zweckmäßigerweise ebenfalls der Absorptionsvorrichtung (2500) zugeführt.

In der beschriebenen Ausführungsform stellen die Einrichtungen (2100), (2200), (2300) und (2500) einzelne (Unter-)Anlagenteile des (Gesamt-)Anlagenteils (2100-2500) aus **B.I**) dar.

Der auf diese Weise erhaltene Phosgen-haltige Prozessabgasstrom (170) enthält den in der Reaktion gebildeten Chlorwasserstoff und wird daher der Chlorwasserstoffabtrennung in der Abtrenneinheit (2600) zugeführt. Diese **Abreicherung des Chlorwasserstoffgehalts aus B.II**) erfolgt bevorzugt durch Absorption von Chlorwasserstoff in Wasser oder verdünnter Salzsäure als Absorptionsmittel (180) in einer weiteren Absorptionsvorrichtung (2600; "HCl-Absorptionskolonne"), wobei ein Salzsäure enthaltender Strom (190) und, bevorzugt nach Durchlaufen eines Brüdenkondensators (2630), ein gasförmiger, Lösungsmittel und ggf. gasförmige Nebenkomponenten enthaltender Phosgen-haltiger Prozessabgasstrom (200) erhalten werden. Dieser Schritt kann nach einem beliebigen aus dem Stand der Technik bekannten Verfahren erfolgen. Bevorzugt sind Verfahrensweisen, wie in EP 2 021 275 B1 beschrieben.

Als Absorptionsmittel (180) wird Wasser (z. B. Dampfkondensat) oder Salzsäure einer Konzentration im Bereich von 0,50 Massen-% bis 15,0 Massen-% (verdünnte Salzsäure) eingesetzt. Der Salzsäure enthaltende Strom (190) wird in den Salzsäuretank (2610) abgelassen. Neben dem Salzsäuretank (2610) ist ein weiter Lagertank (2640) so angeordnet, dass er bei Bedarf Strom (190) aufnehmen kann. Dieser sog. "Dünnsäuretank" (2620) wird dann benutzt, wenn die Zusammensetzung des Stroms (190) von der für Salzsäure geforderten Spezifikation signifikant abweicht, was bei einer Produktionsunterbrechung auftritt.

Durch die bei der Absorption des Chlorwasserstoffs freiwerdende Wärme wird in Strom (170) enthaltendes Lösungsmittel überwiegend bis vollständig in den Gasstrom (200) überführt.

In der bevorzugten Ausführungsform unter Einsatz des Brüdenkondensators (2630) wird in demselben ein flüssiger Strom (191) erhalten. Der Strom (191) enthält im Allgemeinen wässrige und organische Bestandteile. In dieser Ausführungsform kann es daher zweckmäßig sein, den Strom (191) in einer Phasentrennvorrichtung (2640) in eine wässrige Phase (192) und eine organische Phase (193) zu trennen. Die wässrige Phase (192) wird bevorzugt - insbesondere bevorzugt nach Ablassen in den Dünnsäuretank (2620) - als Bestandteil des Absorptionsmittels (180) in die HCl-Absorptionskolonne (2600) zurückgeführt. Die wassergesättigte organische Phase (193) wird zur weiteren Verwendung in einem Vorlagebehälter (2650) gesammelt und bevorzugt nach Trocknung (insbesondere über Molsiebe in einem Trocknungsbehälter 2660) rezykliert, vorzugsweise in die Destillationskolonne (2200), bspw. indem die organische Phase mit dem Strom (80) vermischt oder wie in FIG. 3 gezeigt unabhängig von Strom (80) in diese Destillationskolonne eingeführt wird.

Zur **Reindarstellung des gewünschten Isocyanats (1)** ist es bevorzugt, die nach Abtrennung des Lösungsmittels in der oben beschriebenen Lösungsmittelkolonne (2200) erhaltene Flüssigphase (100) in einer Destillationseinheit (2400) **(B.III))** weiter zu destillieren. Dabei wird aus der Flüssigphase (100) ein flüssiger Isocyanat-Strom (140) gewonnen, wobei ein Nebenkomponenten und ggf. Lösungsmittel enthaltender gasförmiger Strom (150) anfällt. Optional umfasst B.III) die Abtrennung von polymeren Isocyanatfraktionen in einer vorgeschalteten Einrichtung zur Polymerabtrennung (2410) als Strom (141). Die destillative Reinigung der Flüssigphase (100) kann nach einem beliebigen aus dem Stand der Technik bekannten Verfahren erfolgen. Geeignete Verfahren sind beschrieben in EP1475367B1 oder auch in EP 1 371 635 B1.

Der Strom (140) umfasst also in dieser Ausführungsform das Isocyanat (1) in einer gereinigten Form. Dabei kann (140) auch summarisch für verschiedene Isocyanat-Ströme (1) *unterschiedlicher Isomerenzusammensetzung* (140-1, 140-2, ...) stehen, sofern die Destillation in 2400 nicht nur eine Reinigung, sondern auch eine Isomerentrennung beinhaltet. Daneben enthält auch der in besonderen Ausführungsformen anfallende Strom 141 Isocyanate (1), wobei Strom 141 vor allem *polymere Isocyanatfraktionen* (das sind Isocyanate (1), die sich von polymerisierten Aminen ableiten lassen, z. B. Polymethylenpolyphenylenpolyisocyanate mit drei oder mehr Benzol-"Kernen") umfasst. Auch der in besonderen Ausführungsformen - d. h. insbesondere bei Verzicht auf die vorgeschaltete Polymerabtrennung in 2410 - anfallende "Rückstandsstrom" 143 enthält noch Isocyanat (1), welches aus diesem Strom gewonnen werden kann. Die letztgenannte Ausführungsform eignet sich besonders für die Herstellung von Toluylendiisocyanat, wobei dann die Destillationsvorrichtung 2400 vorzugsweise als Trennwandkolonne ausgestaltet wird.

Die Destillationsvorrichtungen (2200), (2300), sofern vorhanden (2410) und (2400), werden bevorzugt bei gegenüber Umgebungsdruck vermindertem Druck betrieben. Die dabei entstehenden Prozessabgasströme werden, gegebenenfalls nach einer Lösungsmittelabtrennung durch Kondensation oder Tropfenabscheidung, der ersten Phosgenzersetzungseinrichtung (3011) zugeführt.

Der auf diese Weise erhaltene Phosgen-haltige Prozessabgasstrom (200) wird der **ersten Phosgenzersetzungseinrichtung (3011) aus C**) zugeführt. Hierbei wird Phosgen katalytisch, bevorzugt an Aktivkohle, unter Verwendung eines wässrigen Stroms (260) zersetzt, wobei ein gasförmiger, gegebenenfalls Lösungsmittel und gegebenenfalls gasförmige Nebenkomponenten enthaltender Strom und ein salzsaurer flüssiger Strom erhalten werden, der teilweise in den Tank für verdünnte Salzsäure (2620) ausgeschleust und teilweise rezykliert und als Bestandteil des wässrigen Strom (260) eingesetzt wird. Bevorzugt werden das Prozessabgas und der wässrige Strom (260) im Gleichstrom durch das Aktivkohlebett geführt. Der gasförmige Strom durchläuft gegebenenfalls eine Adsorptionsvorrichtung (3020) zur Adsorption von Lösungsmittel (nicht gezeigt in FIG. 3) unter Erhalt des Prozessabgasstroms (210).

Der so erhaltene von Phosgen befreite Prozessabgasstrom (210) wird der **Verbrennungseinrichtung (6000) aus D**) zugeführt.

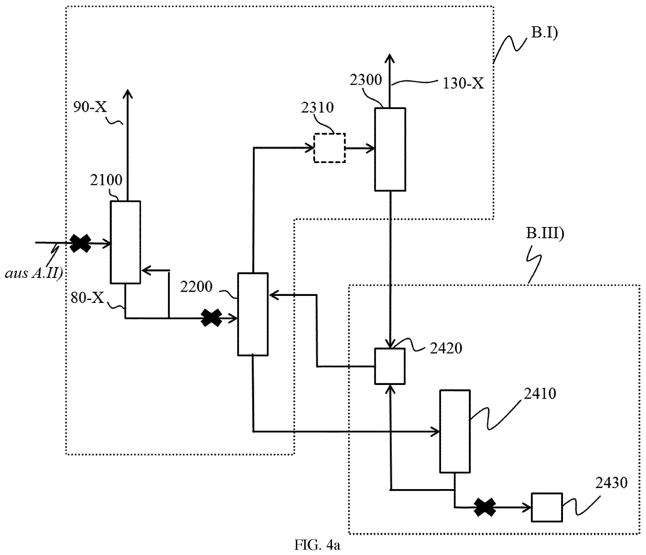

Bevorzugte **Ausführungsformen der erfindungsgemäßen Kreislauffahrweisen des in** **FIG. 3** **gezeigten Prozesses bei einer Unterbrechung der Isocyanatproduktion** sind in **FIG.4a****-b** dargestellt:
Eine **erste Kreislauffahrweise** wird aufgebaut, indem das Destillat der Destillationsvorrichtung (2200), welches überwiegend aus Lösungsmittel (4) besteht, in die Destillationsvorrichtung (2300) gefahren wird. Der Sumpf der Lösungsmittel-Reinigung (2300) wird gekühlt (nicht gezeigt in den Abbildungen) und läuft in einen Lagertank (2420) (den sog. "MDI-Rohware-Lagertank"; vgl. **FIG. 4a****,** in FIG. 3 nicht gezeigt). Dabei wird die Lösungsmittel-Reinigung in der Destillationsvorrichtung (2300) weiter betrieben. Das sich mit Lösungsmittel verdünnende Roh-Isocyanat aus dem MDI-Rohware-Lagertank (2420) wird von dort mittels Austragepumpe zurück in die Lösungsmittel-Destillation (2200) gefahren und so eine Kreislauffahrweise (2200) → (2300) → (2200) eingestellt.

Eine **zweite Kreislauffahrweise** wird aufgebaut, indem die Isocyanat-Rohlösung aus der Lösungsmittel-Destillation (2200) über die Einrichtung zur Abtrennung polymerer Isocyanat-Fraktionen (2410) insbesondere kalt (d. h. bei abgeschalteter Sumpfbeheizung der Kolonne (2410)) in den MDI-Rohware-Lagertank (2420) und von dort zurück in die Lösungsmittel-Destillation (2200) gefahren und so eine Kreislauffahrweise (2200) → (2410) → (2200) eingestellt wird; vgl. ebenfalls **FIG. 4a****.** Solange die Lösungsmittelreinigung (2300) in Betrieb ist, wird deren Destillat (130-X; Lösungsmittel (4) mit Spuren an Phosgen (3)) in den Phosgenabsorber (2500) gepumpt und dort gesammelt. Erforderlichenfalls werden zu große Mengen aus dem Phosgenabsorber (2500) in den Phosgenlösungsbehälter (1030) geleitet. Die erste und die zweite Kreislauffahrweise haben die Leitung vom Lagertank (2420) in die Destillationsvorrichtung (2200) gemeinsam.

Eine **dritte Kreislauffahrweise** wird etabliert, indem die der Entphosgenierkolonne (2100) entnommene Flüssigphase (80-X) enthaltend Roh-Isocyanat-Lösung im Kreis zurück in die Kolonne gepumpt wird; vgl. ebenfalls **FIG. 4a****.** Vorzugsweise geschieht dies mittels eines mit Dampf beheizten Umlaufverdampfers (nicht gezeigt in FIG. 4a).

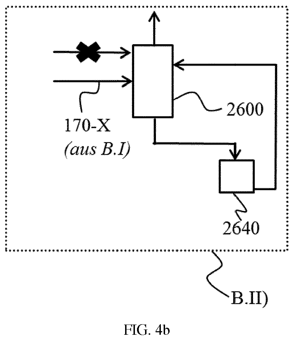

Eine **vierte Kreislauffahrweise** wird etabliert, indem der Ablauf der Chlorwasserstoff-Absorption (2600) vom Salzsäure-Tank (2630) auf den schwachen Salzsäure-Tank (2640) umgestellt wird. Von dort wird die schwache Salzsäure zurück zum Kopf des Absorbers der Chlorwasserstoff-Absorption gefahren und auf diese Weise eine Kreislauffahrweise (2600) → (2640) → (2600) eingestellt; vgl. **FIG. 4b****).** Die Zufuhr weiteren Absorptionsmittels (180) wird dabei unterbrochen (gezeigt durch das "**x**" in der zugehörigen Leitung), und der Abgasweg aus B.I) über B.II) in die Phosgenzersetzung aus C) bleibt offen.

Sind alle vorgenannten Kreislauffahrweisen etabliert, fließen die gasförmigen Austrittströme der Entphosgenierung (90-X) und der Lösungsmitteldestillation (130-X) durch die Phosgenabsorption (B.I), 2500) und von da aus als Strom (170-X) in die Chlorwasserstoffabsorption (B.II), 2600). Dabei verbleibt die Phosgenabsorption (B.I), 2500) vorzugsweise bei ihrem üblichen Betriebsdruck und der üblichen Betriebstemperatur im Regelbetrieb; lediglich die Einspeisung von Lösungsmittel (4) unterbleibt.

Die zuvor genannten Kreislauffahrweisen eins bis vier eignen sich besonders, wenn wie in FIG. 2 dargestellt die Reaktionsstrecke (1000) aus A) außer Betrieb genommen werden soll.

Die Erfindung betrifft den Fall, dass die Produktion des chemischen Produkts unterbrochen werden muss, da in einem Anlagenteil eine Maßnahme durchgeführt werden muss, die zumindest zeitweise mit dem Anfall Sauerstoff-haltigen Abgases (400-X) verbunden ist. Sauerstoff kann beispielsweise eindringen, wenn ein außer Betrieb genommener Anlagenteil geöffnet werden muss oder in einem Anlagenteil eine Leckage aufgetreten ist. Die so entstehenden Sauerstoff-reichen Abgasströme (400-X) werden erfindungsgemäß der mindestens einen **zweiten Phosgenzersetzungseinrichtung (3012)** zugeführt. Deren Betriebsweise kann prinzipiell genauso erfolgen wie zuvor für die erste Phosgenzersetzungseinrichtung (3011) beschrieben, worauf an dieser Stelle verwiesen wird. Die Phosgenzersetzungseinrichtung (3012) umfasst bevorzugt einen oder mehrere (3012-1, 3012-2, ...), mit Aktivkohle (z. B. Norit RB4C) befüllte Phosgenzersetzungs-Teilanlagen (Phosgenzersetzungsreaktoren, sog. Phosgenvernichtungstürme), die bei gegenüber Umgebungsdruck vermindertem Druck, insbesondere bei einem Druck betrieben werden (siehe die Beschreibung der ersten Phosgenzersetzungseinrichtung (3011)). Dabei kann die zweite Phosgenzersetzungseinrichtung (3012) auch mehrere, insbesondere zwei, Phosgenzersetzungs-Teilanlagen (3012-1, 3012-2), die parallel geschaltet sind, umfassen. Diese Phosgenzersetzungs-Teilanlagen (3012-1, 3012-2) werden mit Sauerstoff-reichem Abgas aus unterschiedlichen Quellen gespeist (beispielsweise kann eine Phosgenzersetzungs-Teilanlage (3012-1) für auch im Regelbetrieb anfallendes Sauerstoff-reiches Abgas (410-X), das insbesondere bei Probenentnahmen für analytische Zwecke anfällt, vorgehalten werden, werden eine andere Phosgenzersetzungs-Teilanlage (3012-2) nur für bei einer Maßnahme anfallendes Sauerstoff-reiches Abgas (400-X) vorgehalten wird). Die Phosgenzersetzungs-Teilanlagen sind dabei aufgebaut und werden betrieben wie zuvor beschrieben. Es kann sich beispielsweise um Rohrreaktoren handeln.

Die Erfindung betrifft ferner eine **Produktionsanlage zur Herstellung eines chemischen Produkts (1)** durch Umsetzung eines H-funktionellen Reaktanten (2) mit Phosgen (3), die ausgestaltet ist wie weiter oben bereits beschrieben und die dazu geeignet ist, mit dem erfindungsgemäßen Verfahren betrieben zu werden. Sämtliche zuvor im Zusammenhang mit dem erfindungsgemäßen Verfahren beschriebenen bevorzugten Ausgestaltungen, insbesondere solche, die bevorzugte *vorrichtungstechnische* Ausgestaltungen betreffen, gelten selbstverständlich auch für die erfindungsgemäße Produktionsanlage.

Durch das erfindungsgemäße Verfahren ergeben sich mindestens die folgenden **Vorteile:**
I) Minimierung der sicherheitstechnischen Einrichtungen und apparatetechnischen Anforderungen durch sichere Einhaltung von Explosionsgrenzen;
II) Die gezielte Zufuhr Sauerstoff-reichen Abgases in die Verbrennungseinrichtung ohne die Notwendigkeit exzessiver Verdünnung mit Inertgasen verringert oder vermeidet das Zuspeisen von Erdgas in die Verbrennungseinrichtung während einer Produktionsunterbrechung;
III) Reduzierung des Zeitaufwands für Reparaturen und Instandhaltungsmaßnahmen, da die Installation einer Stickstoffversorgung zur Inertisierung des betroffenen Bereiches entfällt;
IV) Vermeidung von Austreten von Umweltgiften bei der Probenahme;
V) Vermeidung von Ablagerungen, die bei Zusammenführung von Prozessabgas und Sauerstoff-reichem Abgas infolge von Reaktionen des chemischen Produktes mit Sauerstoff und/oder Feuchtigkeit entstehen können (z. B. Harnstoffbildung im Fall von Isocyanaten);
VI) Vermeidung von Korrosionsschäden in der Produktionsanlage, weil der Kontakt von Chlorwasserstoff und/oder Phosgen mit Feuchtigkeit verhindert wird;
VII) Minimierung des Verbrauchs an Inertgasen wie Stickstoff, weil bei getrennter Führung von Sauerstoff-armem und Sauerstoff-reichem Abgas nicht das gesamte Abgas durch Verdünnung mit Inertgasen aus dem explosionsfähigen Bereich gehalten werden muss; dadurch bedingt
VIII) Reduzierung des Erdgasverbrauchs in der Verbrennungseinrichtung;
IX) Verringerung des Zeitbedarfs für eine Reparatur oder Instandhaltungsmaßnahme.

Der Erfolg der erfindungsgemäßen Vorgehensweise zur separaten Aufarbeitung des Sauerstoff-reichen Abgases durch Einsatz einer zweiten Phosgenzersetzungseinrichtung gegenüber der Nutzung der bereits vorhandenen Phosgenzersetzungseinrichtung in der Herstellung chemischer Produkte durch Umsetzung von Phosgen mit H-funktionellen Reaktanten für den Fachmann überraschend. Die mindestens eine zweite Phosgenzersetzungseinrichtung für Sauerstoff-reiches Abgas ermöglicht eine deutliche Verkürzung von Reparatur- und Instandhaltungsarbeiten und führt ferner zur Verbesserung der Probenahme für analytische Zwecke unter Arbeitssicherheit- und Umweltgesichtspunkten. Die unvermeidbar höheren Investitionskosten, die mit der erfindungsgemäßen Vorgehensweise verbunden sind, werden dadurch rasch kompensiert, was für den Fachmann nicht zu erwarten war. Der zusätzliche Instandhaltungsaufwand ist gering (so ist ein Wechsel der in der zweiten Phosgenzersetzungseinrichtung vorzugsweise eingesetzten Aktivkohle in der Regel nur einmal in fünf Jahren erforderlich).

Die Erfindung wird nun anhand der Beispiele noch näher erläutert.

### Beispiele

### A. Allgemeine Bedingungen

### A.I. Allgemeine Bedingungen für die Herstellung eines Gemisches aus Methylendiphenylendiisocyanat und Polymethylenpolyphenylenpolyisocanat (summarisch MDI) durch Phosgenierung eines Gemisches aus Methylendiphenylendiamin und Polymethylenpolyphenylenpolyamin (summarisch MDA) im Regelbetrieb - vgl. auch FIG. 3

Es wird grundsätzlich verfahren wie in WO 2017/050776 A1 (S. 35 und 36) beschrieben, mit folgenden Unterschieden bzw. weiteren Details der Verfahrensführung, die in WO 2017/050776 A1 nicht explizit erwähnt sind:
- 20,4 t/h MDA als Einsatzstoff werden in 55,0 t/h Monochlorbenzol (MCB9 zu einer 27,1%igen MDA-Lösung gemischt.
- Es werden stündlich 100 t Phosgenlösung mit der MDA-Lösung vermischt.
- Als Sumpfprodukt (100) fallen 25,45 t/h MDI an.
- Die Chlorwasserstoffabsorption (Verfahrensstufe B.II); in WO 2017/050776 A1 *Verfahrensstufe VII*) wird mit den zusätzlichen, zuvor im Zusammenhang mit FIG. 3 erläuterten, Einrichtungen (2630), (2640), (2650) und (2660) betrieben.
- Die Phosgenzersetzungseinrichtung besteht aus zwei mit jeweils 14 m³ Aktivkohle (Norit RB4C) befüllten hintereinandergeschalteten Phosgenvernichtungstürmen, die bei einem vermindertem Druck von 930 mbar (absolut) betrieben werden und in denen Phosgen durch Reaktion mit Wasser (Kondensatwasser und verdünnte Salzsäure) zersetzt wird. Zwischen der Phosgenzersetzungseinrichtung (3011) und der Abgasverbrennung (6000) befindet sich eine Lösungsmitteladsorptionstufe (3021; nicht gezeigt in FIG. 3) als weiterer Bestandteil der Abgasaufarbeitung (C, 3000), in welcher durch Adsorption an Aktivkohle restliches Lösungsmittel (4) aus dem Prozessabgasstrom entfernt wird.
- Abgase werden mittels Ventilatoren, die einen verminderten Druck von 930 mbar (absolut) erzeugt, in die Abgasaufarbeitung gesaugt. Solche Ventilatoren befinden sich hinter der Phosgenzersetzung und hinter der Lösungsmitteladsorption.

### A.II. Allgemeine Bedingungen für die Herstellung von Phosgen (Verfahrensstufe 0) - vgl. auch FIG. 3

Es wird grundsätzlich verfahren wie in WO 2017/050776 A1 beschrieben (S. 36 und 37; in der zugehörigen Abbildung als *Verfahrensstufe IX* bezeichnet), mit folgenden Unterschieden bzw. weiteren Details der Verfahrensführung, die in WO 2017/050776 A1 nicht explizit erwähnt sind:
- Als Ausgangsstoffe werden 4400 Nm³/h Chlor (310) und 4650 Nm³/h Kohlenstoffmonoxid (300) eingesetzt.
- Die Rohrbündel-Phosgengeneratoren enthalten jeweils 10 Tonnen Aktivkohle (Norit RB4C).
- Es werden 42,0 t/h Phosgen in den Phosgenlösungstank (1030) geführt.
- Das Kopfprodukt (320) des zweiten Phosgenverflüssigers, 150 m³/h überschüssiges Kohlenstoffmonoxid und Spuren an Phosgen (0,50 % der Gesamtabgasmenge), wird vor Einleitung in die Abgasaufarbeitung (3000) in einer Absorptionskolonne (sog. "Phosgenwäscher" (4010)), die mit -17 °C kaltem Lösungsmittel (MCB) betrieben wird, vorgereinigt, wobei ein Teil des Phosgens aus dem Gasstrom herausgewaschen wird. Die so erhaltene Phosgen-haltige MCB-Lösung wird der Phosgenabsorption (2500) als flüssiger Strom (340) zugeführt.
- Zur Kälteerzeugung in den Phosgenverflüssigern werden sog. Ammoniakschraubenverdichter eingesetzt. Damit wird das Lösungsmittel MCB, das in der Reaktion Verwendung findet, und das Kühlmittel MCB, mit dem die Kühler für die Kondensation der Abgasströme versorgt werden, auf -17 °C abgekühlt.

### B. Beispiele

### Beispiel 1 (Vergleichsbeispiel): Kurzstillstand einer MDI-Produktionsanlage, umfassend eine Komplettabstellung der Produktionsanlage (mit Ausnahme der Abgasaufarbeitung (C, 3000) und der Abgasverbrennung (D, 6000) sowie von Hilfssystemen wie der Stickstoffversorgung), Durchführung einer Reparaturmaßnahme und Wiederinbetriebnahme der Produktionsanlage.

Die Produktionsanlage wurde wie unter A beschrieben betrieben. Abweichend von FIG. 3 wurden sämtliche Abgase, Prozessabgase und Sauerstoff-reiche Abgase (im Regelbetrieb sind Letztgenannte lediglich Ströme aus Probenahmestellen, die von Zeit zu Zeit anfallen), durch ein und dieselbe Phosgenzersetzungseinrichtung (3010) geführt. In der Brüdenleitung für den Prozessabgasstrom (70) von einem Phosgenierturm (1200) in die Phosgenabsorption (2500) trat eine Leckage auf, zu deren Behebung wie folgt verfahren wurde:
**Abschaltung der Produktionsanlage (mit Ausnahme der Abgasaufarbeitung (C, 3000) und der Abgasverbrennung (D, 6000) sowie von Hilfssystemen wie der Stickstoffversorgung)**
1. Die Zufuhr von MDA wurde unterbrochen, wodurch die Temperatur in der Mischeinrichtung (1100) sogleich absank. Die Zufuhren von MCB aus dem Tank (1040) und Phosgenlösung aus dem Tank (1030) wurden noch 3 Minuten aufrechterhalten, dann wurden beide Ströme ebenfalls abgestellt. Die im Phosgenierturm verbliebene Reaktionslösung wurde über einen Notablass in einen Notablassbehälter (nicht gezeigt in FIG. 3) entleert. Der Gasraum des Notablassbehälters war an die Phosgenabsorption (B.I), 2500) angeschlossen. Der Druck in den an den Notablassbehälter angeschlossenen Apparaten sank auf 980 mbar (absolut). Durch die Leckage wird in das Abgas, das durch die Phosgenabsorption (B.I), 2500), die Chlorwasserstoffadsorption (B.II, 2600) in die Abgasaufarbeitung (C), 3000) geleitet wird, Sauerstoff eingetragen.
2. Nach Abstellung der MDA-Zufuhr wurde die Phosgenherstellung (0, 4000, 4010) abgestellt und alle Ein- und Ausgänge in dieselbe verschlossen. Dies dauerte 3 Minuten.
3. Eine Stickstoffleitung wurde zu der betroffenen Brüdenleitung gelegt. Die Montage dauerte 15 Minuten. Durch das Einblasen von Stickstoff wurde der Sauerstoffgehalt des Abgases reduziert, und der Druck stieg leicht auf 990 mbar (absolut).
4. Die betroffene Brüdenleitung wurde unter Stickstoffeintrag mit MCB gespült, anschließend wurde anhaftendes MCB durch weiteren Stickstoffeintrag ausgetrieben. Das zur Spülung verwendete MCB wurde in den Notablassbehälter geführt. Dieser Vorgang dauerte 3 Stunden.
5. Nach der Notabstellung der Reaktion unter Ziffer 1 wurden die Entphosgenierkolonne (B.I), 2100), die Phosgenabsorption (B.I), 2500), die Lösungsmittel-destillation (B.I), 2200), die Lösungsmittelreinigung (B.I), 2300) und die MDI-Destillation (B.III), 2400, 2410) **abgestellt,** was 10 Minuten dauerte. Die genannten Apparate waren gefüllt mit Lösungsmittel und MDI mit Ausnahme der Phosgenabsorption (2500), die mit Phosgen und MCB gefüllt war. Die Heizungen der genannten Apparate wurden abgestellt, sodass diese auskühlten. Der Gasweg zur Phosgenabsorption (B.I), 2500) blieb offen.
6. Die Chlorwasserstoffabsorption (B.II), 2600) blieb zunächst in Betrieb. Die im Regelbetrieb stark Chlorwasserstoff-haltige Gasphase (70) verlor rasch an Chlorwasserstoff. Innerhalb von 5 Minuten sank die ankommende Chlorwasserstoffmenge soweit, dass in der Chlorwasserstoffabsorption (B.II), 2600) nur noch schwache Salzsäure entstand, die in einen dafür vorgesehenen Salzsäuretank für schwache Salzsäure (2620) geleitet wurde. 30 Minuten nach Beendigung des Abstellens der Reaktionsstrecke (A), 1000) wurde der Chlorwasserstoffabsorber (2600) abgestellt. Der Weg zum Salzsäuretank (2610) wurde geschlossen. Der Weg zum Dünnsäuretank (2620) blieb offen. Der Gasweg von der Phosgenabsorbtion (B.I), 2500) über die Chlorwasserstoff-absorption (B.II), 2600) in die Abgasaufarbeitung (C), 3000) blieb offen. Die Phosgenzersetzung (C, 3010) und die Lösungsmitteladsorption (C), 3020) blieben in Betrieb.
7. Nach Abschaltung der Chlorwasserstoffabsorption (B.II), 2600) wurde das Vakuumsystem der Destillationsvorrichtungen (2200), (2300), (2410) und (2400) abgeschaltet Anschließend wurden diese Destillationsvorrichtungen mit Stickstoff versetzt, sodass sich Umgebungsdruck einstellte. Diese Vorgänge dauerten insgesamt 1 Stunde.
8. Die Kälteerzeugung zur Abkühlung von MCB wurde innerhalb von 30 Minuten abgestellt.
9. Die gesamte Abgasaufarbeitung (C, 3000) blieb in Betrieb. Nach Abstellung des Vakuumsystems (Ziffer 7) wurde im Phosgenierturm, in der Phosgenabsorption, in der Chlorwasserstoffabsorption und in den jeweiligen Gasleitungen (Brüdenleitungen) vorhandenes Abgas mittels des vorhandenen Ventilators angesaugt, bis sich ein Druck von 930 mbar (absolut) einstellte, was 8 Stunden dauerte.

### Durchführung der Reparaturmaßnahme

Zur Behebung der Leckage der Dichtung in der betroffenen Brüdenleitung des Phosgenierturms wurde eine Dichtung ausgetauscht. Dies geschah unter Beibehaltung des verminderten Drucks von 930 mbar (absolut). Nach Einbau der neuen Dichtung wurde die Brüdenleitung vor der Phosgenabsorption mit einem Ventil geschlossen, um den Unterdruck aufzuheben und eine Dichtigkeitprüfung mit Stickstoff durchzuführen. Danach wurden die Reaktionsstrecke (A, 1000), der Phosgenabsorber (B.I, 2500), der Chlorwasserstoffabsorber (B.III), 2600) sowie die Abgasaufarbeitung (C), 3000) (mit Ausnahme der Lösungsmitteladsorption) mit Stickstoff gespült um auch noch letzte Spuren an Sauerstoff zu entfernen. Der Zeitbedarf für die Reparatur betrug 3 Stunden, das Spülen mit Stickstoff weitere 6 Stunden.

Der Wechsel der defekten Dichtung dauerte insgesamt 18 Stunden.

### Wiederinbetriebnahme der Produktionsanlage

Es wurde wie folgt verfahren:
1. Aus dem Lösungsmitteltank (1040) wurde MCB in die entleerte Reaktionsstrecke gepumpt. Nach 6 Stunden war ein ausreichender Flüssigkeitsstand gegeben (MCB lief aus dem Phosgenierturm über in die Entphosgenierkolonne). Die Entphosgenierkolonne wurde anschließend in Betrieb genommen.
2. Kälte- und Vakuumerzeugung wurden wieder in Betrieb genommen, was 4 Stunden dauerte.
3. Die Lösungsmitteldestillationskolonne (B.I), 2200) und die Lösungsmittelreinigung (B.I), 2300) wurden in dieser Reihenfolge nacheinander in Betrieb genommen.
4. Nach Inbetriebnahme der Lösungsmittelreinigung (B.I), 2300) wurde Lösungsmittel aus Lösungsmitteltank (1040) über die Reaktionsstrecke (A, 1000) und die Entphosgenierkolonne (B.I), 2100) in die Lösungsmitteldestillationskolonne (B.I), 2200) geführt, wobei die Beheizung der Reaktionsstrecke und der Entphosgenierkolonne angestellt wurden. Nun war die Lösungsmitteldestillation betriebsbereit und lief über die Lösungsmittelreinigung, den Lösungsmitteltank, die Reaktionsstrecke und den Entphosgenierer im Kreis. Dieser Vorgang dauerte 8 Stunden.
5. Mit dem Anstellen der Lösungsmitteldestillation und -reinigung fiel eine Lösungsmittel-haltige Gasphase an (im Regelbetrieb Strom 130), die kondensiert und der Phosgenabsorption zugeführt wurde. Der Phosgen-freie Sumpfstrom der Lösungsmittelreinigung (im Regelbetrieb Strom 120) wurde in den Lösungsmitteltank (1040) gepumpt. Diese Vorgänge dauerten 4 Stunden.
6. Sobald der kondensierte Lösungsmittel-haltige Gasstrom aus der Lösungsmittelreinigung ankam, wurden in der Phosgenabsorption (B.I), 2500) die Kühlsysteme für die Kondensation der im Regelbetrieb anfallenden Prozessabgasströme (70) und (90) betriebsbereit gemacht, und der Ablauf der Phosgenabsorption auf den Phosgenlösungstank (1030) eingestellt. Anschließend wurde der Weg vom Phosgenlösungstank (1030) zur Mischeinreichtung (1100) und zum Phosgenierturm (1200) geöffnet.
7. Die Phosgenherstellung (0), 4000, 4010) wurde innerhalb von 45 Minuten angefahren, und die Phosgenkonzentration im Phosgenlösungstank (1030) wurde anschließend sukzessive innerhalb von 6 Stunden auf den gewünschten Wert (35%ig) aufkonzentriert.
8. Der mit Lösungsmittel befüllte Phosgenierturm (1200) wurde mit Hilfe eines Wärmeträgers auf 105 °C aufgeheizt. Sobald die Phosgenlösungskonzentration 25 % erreichte, wurde die MDA-Zufuhr geöffnet. Während des Anfahrens wurde ein stöchiometrischer Überschuss von Phosgen zu MDA von 140 % eingestellt; die Produktionskapazität betrug 15 % des angestrebten Werts von 25,45 t/h MDI. Der MDA-Mengenstrom wurde nach 1 h auf 25 % der angestrebten Produktionskapazität erhöht. Bei Erreichen dieser Last wurde ein stöchiometrischer Überschuss von Phosgen zu MDA von 100 % eingestellt. Die MDA-Konzentration in Lösungsmittel wurde dann auf 28 % eingestellt; die Konzentration an Phosgen in der Phosgenlösung (30) hatte mittlerweile 35 % erreicht.
9. Sobald aus dem Überlauf des Phosgenierturms (1200) der Reaktionsstrecke erstes Roh-MDI, Lösungsmittel und Phosgen in der Entphosgenierkolonne (B.I), 2100) ankamen, wurde diese bei einen Druck von 1,6 bar (absolut) auf eine Solltemperatur im Destillationssumpf von 157 °C im Entphosgenierersumpf eingestellt und war damit in Betrieb.
10. Sobald erster Chlorwasserstoff nach dem Start der Phosgenierung seinen Weg über die Phosgenabsorption (B.I), 2500) in die Chlorwasserstoffabsorption (B.II), 2600) fand, wurde Salzsäurekonzentration im Ablauf des Chlorwasserstoffabsorbers (2600) auf 31 % eingestellt. Der Weg der Salzsäure zum Salzsäuretank (2610) wurde geöffnet und der Weg zum Dünnsäuretank (2620) wurde geschlossen. Nun war die Chlorwasserstoff-Absorption in Betrieb. Dieser Vorgang dauerte 2 Stunden und lief parallel zur Inbetriebnahme der Phosgenierung.
11. Sobald der Druck am Kopf der zweiten Lösungsmitteldestillationskolonne (B.I), 2300; in FIG. 3 ist aus Gründen der zeichnerischen Vereinfachung nur eine Kolonne gezeigt) bei 70 mbar (absolut) eingeregelt war und eine Sumpftemperatur von 120 °C erreicht wurde, wurde der Sumpf dieser zweiten Lösungsmitteldestillationskolonne auf den Einlauf der Destillationsvorrichtung zur Abtrennung polymerer Isocyanatfraktionen (B.I), 2410) umgeschaltet. Das polymere Sumpfprodukt wurde in einen MDI-Produkttank gepumpt. Das monomere MDI, das am Kopf der Kolonne anfiel, wurde in weiteren Kolonnen (B.I), 2400) gereinigt.
12. Nun lief die MDI-Anlage mit 25 % der angestrebten Produktionskapazität, und die Last wurde sukzessiver weiter erhöht. Die angestrebte Produktionskapazität von 25,45 t/h MDI wurde erst eingestellt, als in der Destillation (B.III), 2410, 2400) spezifikationsgerechtes Produkt erhalten wurde. Dies dauerte 12 Stunden.

### Bilanz des Energie-, Hilfsstoff- und Zeitbedarfes für das Ab- und Anfahren der Anlage inklusive der Behebung der Leckage:

Der gesamte Zeitbedarf für die Maßnahme bis die Produktionsanlage wieder spezifikationsgerechtes Produkt mit der angestrebten Kapazität von 25,45 t/h MDI lieferte, betrug 64 Stunden. Dadurch wurde die Produktionsmenge um 1629 Tonnen MDI verringert. Der Stickstoffverbrauch während der Reparaturmaßnahme (3 Stunden) und während der Inertisierung der Anlage mit Stickstoff (6 Stunden) betrug 450 Nm³. Der Erdgasverbrauch für die Abgasverbrennung (D), 6000) betrug während der Maßnahme 8450 Nm³.

### Beispiel 2 (erfindungsgemäß): Kurzstillstand einer MDI-Produktionsanlage, umfassend eine Teilabstellung der Produktionsanlage, die Etablierung von Kreislauffahrweisen für nicht abgestellte Anlagenteile, die Durchführung einer Reparaturmaßnahme und Wiederinbetriebnahme der Produktionsanlage.

Die Produktionsanlage wurde wie unter A beschrieben betrieben. Wie in FIG. 3 gezeigt, waren zwei (gleich aufgebaute) Phosgenzersetzungseinrichtungen (3011, 3012) vorhanden. Die erste Phosgenzersetzungseinrichtung (3011) dient ausschließlich zur Aufnahme von Prozessabgas. Die zweite Phosgenzersetzungseinrichtung (3012) wird nur für Sauerstoff-haltige Abgasströme verwendet. Zwischen der zweiten Phosgenzersetzungseinrichtung (3012) und der Abgasverbrennung ist keine Lösungsmitteladsorptionsstufe zwischengeschaltet. Im Regelbetrieb sind dies lediglich Ströme aus Probenahmestellen, die von Zeit zu Zeit anfallen; die Einrichtung (3012) wurde aber permanent betriebsbereit gehalten. Ein Rohrleitungsnetz aus elektrisch leitfähigem Polyethylen, aufweisend absperrbare Anschlüsse für bewegliche Schläuche, stand in allen Bereichen Produktionsanlage zur Verfügung, um Sauerstoff-reiche Abgase in diese zweite Phosgenzersetzungseinrichtung (3012) zu führen. Prozessabgas (210-X) aus der ersten Phosgenzersetzungseinrichtung (3011) und Sauerstoff-haltiges Abgas (410-X) aus der zweiten Phosgenzersetzungseinrichtung (3012) werden getrennt voneinander in die Abgasverbrennung (D, 6000) geführt und dort verbrannt.

In der Brüdenleitung für den Prozessabgasstrom (70) von einem Phosgenierturm (1200) in die Phosgenabsorption (2500) trat eine Leckage auf, zu deren Behebung wie folgt verfahren wurde:
1. Der Gasaustritt des Phosgenierturms (1200), im Regelbetrieb für Strom (70), wurde über das festangeschlossene Rohrleitungsnetz durch Öffnen eines Ventils zur zweiten Phosgenzersetzungseinrichtung (3012) geöffnet. Verbindungen von der Reaktionsstrecke (A, 1000) mit den übrigen Teilen der Produktionsanlage, die im Regelbetrieb verwendet werden, wurden durch das Schließen der entsprechenden Ventile geschlossen. Der Druck im Phosgenierturm sank auf 930 mbar (absolut). An der Leckagestelle (die zwischen dem Austritt aus dem Phosgenierturm (1200) und dem - nun verschlossenen - Ventil in der Leitung für Strom (70) des Regelbetriebs lag) wurde ein Schlauch angebracht, mittels dessen eine Verbindung zur zweiten Phosgenzersetzungseinrichtung (3012) hergestellt wurde; auch hier stellte sich ein Druck von 930 mbar (absolut) ein.
2. Die Zufuhr von MDA wurde unterbrochen, wodurch die Temperatur in der Mischeinrichtung (1100) sogleich absank. Die Zufuhr von MCB aus dem Tank (1040) wurde noch 3 Minuten aufrechterhalten, Phosgenlösung aus dem Tank (1030) wurde noch 30 Sekunden weiter zugeführt, dann wurden beide Ströme ebenfalls abgestellt. Die im Phosgenierturm verbliebene Reaktionslösung wurde über einen Notablass in einen Notablassbehälter (nicht gezeigt in FIG. 3) entleert, was 15 Minuten dauerte. Dieser Notablassbehälter war an die erste Phosgenzersetzungseinrichtung (3011) angeschlossen. Nach der Entleerung des Phosgenierturmes wurde der Abgasweg zur ersten Phosgenzersetzungseinrichtung (3011), der über die Phosgenabsorption (B.I), (2500) führt, direkt hinter dem Gasaustritt des Notablassbehälters geschlossen. Der Notablassbehälter war nun nicht mehr an die Phosgenabsorption (B.I), 2500) angeschlossen, sondern wurde über eine fest angeschlossene Abgasleitung durch Öffnen eines Ventils an die zweite Phosgenzersetzungseinrichtung (3012) angeschlossen, um Sauerstoff-haltiges Abgas (400-X) dorthin abzuführen (siehe auch FIG. 2, wo jedoch der Notablassbehälter aus Gründen der zeichnerischen Vereinfachung weggelassen wurde).
3. Nach Abstellung der MDA-Zufuhr wurde die Phosgenherstellung (0, 4000, 4010) abgestellt und alle Ein- und Ausgänge in dieselbe verschlossen. Dies dauerte 3 Minuten.
4. Die betroffene Brüdenleitung wurde mit MCB gespült, anschließend wurde anhaftendes MCB durch Stickstoffeintrag ausgetrieben. Das zur Spülung verwendete MCB wurde in den Notablassbehälter geführt.
5. Nach der Notabstellung der Reaktion unter Ziffer 1 wurden die Entphosgenierkolonne (B.I), 2100), die Lösungsmitteldestillation (B.I), 2200), die Lösungsmittel-reinigung (B.I), 2300), die Einrichtung zur Polymerabtrennung (B.III), 2410), die Destillationsvorrichtung (B.III), 2400) und die Chlorwasserstoffabsorption (B.II), 2600) **in Kreislauffahrweise gestellt.** Dies geschah wie weiter oben anhand der Abbildungen FIG. 4a und FIG. 4b erläutert. Die gasförmigen Austrittströme der Entphosgenierung (90-X) und der Lösungsmitteldestillation (130-X) flossen dabei durch die Phosgenabsorption (B.I), 2500) und von da aus als Strom (170-X) in die Chlorwasserstoffabsorption (B.II), 2600). Dabei verblieb der Phosgenabsorption (B.I), 2500) bei ihrem üblichen Betriebsdruck (1,6 bar (absolut)); lediglich Einspeisung von Frisch-MCB aus dem Lösungsmitteltank (1040) auf den Kopf der Phosgenabsorption wurde geschlossen. Im Gegensatz zum Vergleichsbeispiel wurde das Vakuumsystem der Destillationsvorrichtungen (2200), (2300), (2410) und (2400) in Betrieb gehalten.
6. Die Kälteerzeugung zur Abkühlung von MCB wurde ebenfalls in Betrieb gehalten.
7. Die gesamte Abgasaufarbeitung (C, 3000) blieb in Betrieb. Von Einleitung der Notabstellung bis zur vollständigen Vorbereitung der Anlage auf die Instandhaltungsmaßnahme vergingen insgesamt 198 Minuten.

### Durchführung der Reparaturmaßnahme

Zur Behebung der Leckage der Dichtung in der betroffenen Brüdenleitung des Phosgenierturms wurde eine Dichtung ausgetauscht. Dies geschah unter Beibehaltung des verminderten Drucks von 930 mbar (absolut). Nach Einbau der neuen Dichtung wurde die Verbindung zur zweiten Phosgenzersetzungseinrichtung (3012) geschlossen, um den Unterdruck aufzuheben und eine Dichtigkeitsprüfung mit Stickstoff durchzuführen. Danach wurde die Reaktionsstrecke (A, 1000) mit Stickstoff gespült um auch noch letzte Spuren an Sauerstoff zu entfernen. Der Zeitbedarf für die Reparatur betrug 3 Stunden, das Spülen mit Stickstoff 1 Stunde.

Der Wechsel der defekten Dichtung dauerte insgesamt 7,3 Stunden.

### Wiederinbetriebnahme der Produktionsanlage

Es wurde wie folgt verfahren:
1. Aus dem Lösungsmitteltank (1040) wurde MCB in die entleerte Reaktionsstrecke gepumpt. Nach 6 Stunden war ein ausreichender Flüssigkeitsstand gegeben (MCB lief aus dem Phosgenierturm über in die Entphosgenierkolonne). Die Entphosgenierkolonne wurde anschließend in Betrieb genommen.
2. Die Phosgenherstellung (0), 4000, 4010) wurde angefahren, und die Phosgenkonzentration im Phosgenlösungstank (1030) wurde sukzessive innerhalb von 6 Stunden auf den gewünschten Wert (35%ig) aufkonzentriert.
3. Der mit Lösungsmittel befüllte Phosgenierturm (1200) wurde mit Hilfe eines Wärmeträgers auf 105 °C aufgeheizt. Sobald die Phosgenlösungskonzentration 25 % erreichte, wurde die MDA-Zufuhr geöffnet. Während des Anfahrens wurde ein stöchiometrischer Überschuss von Phosgen zu MDA von 140 % eingestellt; die Produktionskapazität betrug 15 % des angestrebten Werts von 25,45 t/h MDI. Der MDA-Mengenstrom wurde nach 1 h auf 25 % der angestrebten Produktionskapazität erhöht. Bei Erreichen dieser Last wurde ein stöchiometrischer Überschuss von Phosgen zu MDA von 100 % eingestellt. Die MDA-Konzentration in Lösungsmittel wurde dann auf 28 % eingestellt; die Konzentration an Phosgen in der Phosgenlösung (30) hatte mittlerweile 35 % erreicht.
4. Sobald aus dem Überlauf des Phosgenierturms (1200) der Reaktionsstrecke erstes Roh-MDI, Lösungsmittel und Phosgen in der Entphosgenerkolonne (B.I), 2100) ankamen, wurde diese bei einen Druck von 1,6 bar (absolut) auf eine Solltemperatur im Destillationssumpf von 157 °C im Entphosgenierersumpf eingestellt und war damit in Betrieb.
5. Sobald erster Chlorwasserstoff nach dem Start der Phosgenierung seinen Weg über die Phosgenabsorption (B.I), 2500) in die Chlorwasserstoffabsorption (B.II), 2600) fand, wurde Salzsäurekonzentration im Ablauf des Chlorwasserstoffabsorbers (2600) auf 31 % eingestellt. Der Weg der Salzsäure zum Salzsäuretank (2610) wurde geöffnet und der Weg zum Dünnsäuretank (2620) wurde geschlossen. Nun war die Chlorwasserstoff-Absorption in Betrieb. Dieser Vorgang dauerte 2 Stunden und lief parallel zur Inbetriebnahme der Phosgenierung.
6. Sobald der Druck am Kopf der zweiten Lösungsmitteldestillationskolonne (B.I), 2300; in FIG. 3 ist aus Gründen der zeichnerischen Vereinfachung nur eine Kolonne gezeigt) bei 70 mbar (absolut) eingeregelt war und eine Sumpftemperatur von 120 °C erreicht wurde, wurde der Sumpf dieser zweiten Lösungsmitteldestillationskolonne auf den Einlauf der Destillationsvorrichtung zur Abtrennung polymerer Isocyanatfraktionen (B.I), 2410) umgeschaltet. Das polymere Sumpfprodukt wurde in einen MDI-Produkttank gepumpt. Das monomere MDI, das am Kopf der Kolonne anfiel, wurde in weiteren Kolonnen (B.I), 2400) in die gewünschte Zusammensetzung der Isomeren getrennt.
7. Nun lief die MDI-Anlage mit 25 % der angestrebten Produktionskapazität, und die Last wurde sukzessiver weiter erhöht. Die angestrebte Produktionskapazität von 25,45 t/h MDI wurde erst eingestellt, als in der Destillation (B.III), 2410, 2400) spezifikationsgerechtes Produkt erhalten wurde. Dies dauerte 8 Stunden.

### Bilanz des Energie-, Hilfsstoff- und Zeitbedarfes für das Ab- und Anfahren der Anlage inklusive der Behebung der Leckage:

Der gesamte Zeitbedarf für die Maßnahme bis die Produktionsanlage wieder spezifikationsgerechtes Produkt mit der angestrebten Kapazität von 25,45 t/h MDI lieferte, betrug 29 Stunden und 18 Minuten. Dadurch wurde die Produktionsmenge um 746 Tonnen MDI verringert. Der Stickstoffverbrauch während der Reparaturmaßnahme (1 Stunde) und während der Inertisierung der Anlage mit Stickstoff (1 Stunde) betrug 100 Nm³. Der Erdgasverbrauch für die Abgasverbrennung (D), 6000) betrug während der Maßnahme 3211 Nm³.

**Fazit:** Im erfindungsgemäßen Beispiel 2 mit zwei separat installierten Phosgenzersetzungseinrichtungen (3011, 3012) und Kreislauffahrweise der nicht betroffenen Anlagenteile werden 61 % Primärenergie (Dampf und Strom) und ca. 80 % Stickstoff weniger verbraucht als bei einer Komplettabstellung der Anlage wie in Beispiel 1 (Vergleichsbeispiel). Zusätzlich ergibt sich eine stark verbesserte Produktivität der Anlage, da wegen des geringeren Zeitbedarfes für die ganze Aktion (Abfahren, Maßnahme und Anfahren) 908 Tonnen an MDI mehr produziert werden konnten.

## Patentansprüche

1. Verfahren zum Betreiben einer Produktionsanlage zur Herstellung eines chemischen Produkts (1) durch Umsetzung eines H-funktionellen Reaktanten (2) mit Phosgen (3) bei einer Produktionsunterbrechung, wobei die Produktionsanlage folgende Anlagenteile aufweist:
A) eine Reaktionsstrecke (1000) geeignet zur Umsetzung eines H-funktionellen Reaktanten (2) mit Phosgen (3), aufweisend:
A.I) eine Mischzone (1100) zur Vermischung des H-funktionellen Reaktanten (2) und Phosgen (3) zu einem Reaktionsgemisch (50),
A.II) eine mit der Mischzone (1100) verbundene Reaktionszone (1200) zur Umsetzung des in A.I) erhaltenen Reaktionsgemisches (50) unter Ausbildung einer Flüssigphase (60) enthaltend das chemische Produkt (1) sowie Phosgen (3) und eines Phosgen-haltigen Prozessabgasstroms (70);
B) eine mit der Reaktionsstrecke (1000) verbundene Aufarbeitungsstrecke (2000) aufweisend:
B.I) eine Trenneinheit (2100-2500) zur Trennung der in A.II) erhaltenen Flüssigphase (60) in einen Phosgen-haltigen Prozessabgasstrom (170) und in eine Flüssigphase (100) enthaltend das chemische Produkt (1);
C) eine Abgasaufarbeitungsstrecke (3000) geeignet zur Aufarbeitung während der Herstellung des chemischen Produkts (1) und während der Produktionsunterbrechung anfallender Phosgen-haltiger Abgasströme, aufweisend eine erste Phosgenzersetzungseinrichtung (3011) und eine zweite Phosgenzersetzungseinrichtung (3012), wobei die erste und die zweite Phosgenzersetzungseinrichtung getrennt voneinander mit Phosgen-haltigen Abgasströmen anströmbar sind;
D) eine Verbrennungseinrichtung (6000) geeignet zur Verbrennung des in Abgasaufarbeitungsstrecke (3000) anfallenden aufgearbeiteten Abgases;
wobei während der Herstellung des chemischen Produkts (1) in der Reaktionsstrecke (1000) Phosgen (3) im stöchiometrischen Überschuss bezogen auf alle aktiven Wasserstoffatome des H-funktionellen Reaktanten eingesetzt wird, wobei der in der Reaktionszone (1200) aus A.II) erhaltene Phosgen-haltige Prozessabgasstrom (70) und der in der Trenneinheit (2100-2500) aus B.I) anfallende Phosgen-haltige Prozessabgasstrom (170), jeweils optional nach Durchlaufen weiterer Aufarbeitungsschritte, der ersten Phosgenzersetzungseinrichtung (3011) zugeführt werden,
wobei ferner die Produktion des chemischen Produkts (1) zeitweise durch Abstellen der Zufuhr des H-funktionellen Reaktanten (2) unterbrochen wird,
wobei während dieser Produktionsunterbrechung
• mindestens ein Anlagenteil aus A) und/oder B) außer Betrieb genommen und in mindestens einem der nicht außer Betrieb genommenen Anlagenteile ein Ausgangsstrom dieses mindestens einen nicht außer Betrieb genommenen Anlagenteils
(i) in den jeweiligen Anlagenteil oder
(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt und so der jeweilige Anlagenteil in Kreislauffahrweise gebracht wird, wobei in dem mindestens einen in Kreislauffahrweise gebrachten Anlagenteil Prozessabgas und in dem mindestens einen außer Betrieb genommenen Anlagenteil Sauerstoff-haltiges Abgas (400-X) anfällt;
• Prozessabgas aus dem mindestens einen in Kreislauffahrweise gebrachten Anlagenteil in die erste Phosgenzersetzungseinrichtung (3011) geführt wird, wobei die erste Phosgenzersetzungseinrichtung (3011) auch während der Produktionsunterbrechung in Betrieb bleibt;
• Sauerstoff-haltiges Abgas (400-X) aus dem mindestens einen außer Betrieb genommenen Anlagenteil der zweiten Phosgenzersetzungseinrichtung (3012) zugeführt wird, wobei die zweite Phosgenzersetzungseinrichtung (3012) mindestens während der Produktionsunterbrechung in Betrieb ist, wobei
• von Phosgen befreites Prozessabgas (210-X) aus der ersten Phosgenzersetzungseinrichtung (3011) und von Phosgen befreites Sauerstoff-haltiges Abgas (410-X) aus der zweiten Phosgenzersetzungseinrichtung (3012) getrennt voneinander der Verbrennungseinrichtung (6000) an räumlich getrennten Stellen zugeführt und dort verbrannt werden.

2. Verfahren gemäß Anspruch 1, bei welchem das chemische Produkt (1) ein organisches Carbonat, insbesondere ein Polycarbonat ist.

3. Verfahren gemäß Anspruch 1, bei welchem das chemische Produkt (1) ein Isocyanat, insbesondere Toluylendiisocaynat oder ein Gemisch aus Methylendiphenylendiisocyanat und Polymethylenpolyphenylenpolyisocanat, ist.

4. Verfahren gemäß Anspruch 3, bei welchem die Trenneinheit (2100-2500) aus B.I)
• eine Destillationsvorrichtung (2100) zur Trennung des flüssigen Stroms (60) in einen flüssigen, Lösungsmittel und Isocyanat enthaltenden Strom (80) und einen gasförmigen, Phosgen und Chlorwasserstoff enthaltenden Prozessabgasstrom (90);
• eine Destillationsvorrichtung (2200) zur Trennung des flüssigen Lösungsmittel und Isocyanat enthaltenden Stroms (80) in einen Lösungsmittel enthaltenden Prozessabgasstrom (110) und eine flüssigen, Isocyanat enthaltenden Strom (100);
• eine Destillationsvorrichtung (2300) zur Trennung des Lösungsmittel enthaltenden Prozessabgasstroms (110) in einen flüssigen, Lösungsmittel enthaltenden Strom (120) und einen gasförmigen, Phosgen-haltigen Prozessabgasstrom (130)
aufweist.

5. Verfahren gemäß Anspruch 4, bei welchem die Trenneinheit (2100-2500) aus B.I) weiterhin eine Absorptionsvorrichtung (2500) aufweist, in welcher die Phosgen-haltigen Prozessabgasströme (70), (90) und (130) durch Absorption in einem Lösungsmittel (4) unter Erhalt eines flüssigen, Lösungsmittel und Phosgen enthaltenden Stroms (160) und eines gasförmigen, Chlorwasserstoff und Lösungsmittel enthaltenden Prozessabgasstroms (170) gereinigt werden, wobei bevorzugt die gasförmigen Phosgen-haltigen Prozessabgasströme (70) und (90) zunächst vereint werden und der vereinigte Phosgen-haltige Prozessabgasstrom aus (70) und (90) sowie der Phosgen-haltige Prozessabgasstrom (130) jeweils kondensiert und dann flüssig in die Absorptionsvorrichtung (2500) eingetragen werden.

6. Verfahren gemäß einem der Ansprüche 3 bis 5, insbesondere gemäß Anspruch 5, bei welchem die Aufarbeitungsstrecke (2000) aus B) zusätzlich zur Trenneinheit (2100-2500) aus B.I),
B.II) eine Abtrenneinheit (2600) zur Abtrennung von Chlorwasserstoff aus dem Phosgen-haltigen Prozessabgasstrom (170)
aufweist, in welcher der Phosgen-haltige Prozessabgasstrom (170) an Chlorwasserstoff abgereichert wird, wobei, bevorzugt nach Durchlaufen eines Brüdenkondensators (2630), ein gasförmiger, Lösungsmittel und ggf. gasförmige Nebenkomponenten enthaltender Phosgen-haltiger Prozessabgasstrom (200) erhalten wird, wobei der Phosgen-haltige Prozessabgasstrom (200) der ersten Phosgenzersetzungseinrichtung (3011) zugeführt wird.

7. Verfahren gemäß Anspruch 6, bei welchem die Abtrennung des Chlorwasserstoffs in der Abtrenneinheit (2600) durch Absorption von Chlorwasserstoff in Wasser oder Salzsäure einer Konzentration im Bereich von 0,50 Massen-% bis 15,0 Massen-%, bezogen auf die Gesamtmasse der Salzsäure, durchgeführt wird, wobei neben dem Lösungsmittel und ggf. gasförmige Nebenkomponenten enthaltenden Phosgen-haltigen Prozessabgasstrom (200) ein Salzsäure enthaltender Strom (190) erhalten wird.

8. Verfahren gemäß einem der Ansprüche 3 bis 7, bei welchem die Aufarbeitungsstrecke (2000) aus B) zusätzlich zur Trenneinheit (2100-2500) aus B.I) und, sofern vorhanden, zusätzlich zur Abtrenneinheit (2600) aus B.II),
B.III) eine Destillationseinheit (2400) zur Aufarbeitung der Flüssigphase (100) enthaltend das Isoycanat
aufweist, wobei der Destillationseinheit (2400) optional eine Einrichtung zur Abtrennung polymerer Isocyanatfraktionen (2410) vorschaltet ist.

9. Verfahren gemäß Anspruch 8, aufweisend die Einrichtung zur Abtrennung polymerer Isocyanatfraktionen (2410), bei welchem während der Produktionsunterbrechung
• eine erste Kreislauffahrweise ausgehend vom Kopf der Destillationsvorrichtung (2200) über die Destillationsvorrichtung (2300) zurück in die Destillationsvorrichtung (2200);
• eine zweite Kreislauffahrweise ausgehend vom Sumpf der Destillationsvorrichtung (2200) über die Einrichtung zur Abtrennung polymerer Isocyanat-Fraktionen (2410) zurück in die Destillationsvorrichtung (2200);
• eine dritte Kreislauffahrweise ausgehend von der Destillationsvorrichtung (2100) zurück in dieselbe, und
• eine vierte Kreislauffahrweise ausgehend von der Abtrenneinheit (2600) zur Abtrennung von Chlorwasserstoff zurück in dieselbe
etabliert werden, wobei die Reaktionsstrecke (1000) aus A) außer Betrieb genommen wird.

10. Verfahren gemäß einem der vorstehenden Ansprüche, bei welchem die Produktionsanlage über eine Phosgenherstellungsstrecke (0) umfassend eine Vorrichtung (4000) zur Phosgenherstellung aus Kohlenstoffmonoxid (300) und Chlor (310) verfügt, wobei die Herstellung von Phosgen bei der Produktionsunterbrechung, gegebenenfalls zeitversetzt nach Abstellung der Zufuhr des H-funktionellen Reaktanten (2), abgeschaltet wird.

11. Verfahren gemäß einem der vorstehenden Ansprüche, bei welchem die Anlagenteile der Aufarbeitungsstrecke (2000) aus B) mindestens teilweise bei gegenüber Umgebungsdruck vermindertem Druck betrieben werden, wobei der verminderte Druck durch Vakuumerzeugungsanlagen erzeugt wird, in denen Prozessabgasströme anfallen, welche der ersten Phosgenzersetzungseinrichtung (3011) zugeführt werden.

12. Verfahren gemäß Anspruch 11, bei welchem die erste Phosgenzersetzungseinrichtung (3011) mindestens zwei getrennt voneinander betriebene Phosgenzersetzungs-Teilanlagen (3011-1, 3011-2) aufweist, wobei während der Herstellung des chemischen Produkts (1) eine dieser beiden Phosgenzersetzungs-Teilanlagen (3011-1) nur die Prozessabgasströme aus den Vakuumerzeugungsanlagen zugeführt werden, während der anderen Phosgenzersetzungs-Teilanlage (3011-2) alle übrigen Prozessabgasströme zugeführt werden, wobei während einer Produktionsunterbrechung die Vakuumerzeugungsanlagen außer Betrieb genommen werden und mit der Phosgenzersetzungs-Teilanlage (3011-1) verbunden bleiben.

13. Verfahren gemäß einem der vorstehenden Ansprüche, bei welchem während der Produktion des chemischen Produkts Sauerstoff-haltige Abgasströme (410-X) anfallen, wobei die zweite Phosgenzersetzungseinrichtung (3012) für Sauerstoff-haltige Abgasströme auch während der Produktion des chemischen Produkts (1) betrieben wird und ihr die Sauerstoff-haltigen Abgasströme (410-X) zugeführt werden.

14. Verfahren gemäß Anspruch 13, bei welchem die zweite Phosgenzersetzungseinrichtung (3012) mindestens zwei parallel geschaltete Phosgenzersetzungs-Teilanlagen (3012-1, 3012-2) umfasst, wobei einer der Phosgenzersetzungs-Teilanlagen (3012-1) nur die während der Produktion des chemischen Produkts anfallenden Sauerstoff-haltigen Abgasströme (410-X) zugeführt werden, während der anderen Phosgenzersetzungs-Teilanlage (3012-2) die bei einer Produktionsunterbrechung in dem mindestens einen außer Betrieb genommenen Anlagenteil anfallenden Sauerstoff-reichen Abgasströme (400-X) zugeführt werden.

15. Produktionsanlage zur Herstellung eines chemischen Produkts (1) durch Umsetzung eines H-funktionellen Reaktanten (2) mit Phosgen (3), aufweisend folgende Anlagenteile:
A) eine Reaktionsstrecke (1000) geeignet zur Umsetzung eines H-funktionellen Reaktanten (2) mit Phosgen (3), aufweisend:
A.I) eine Mischzone (1100) zur Vermischung des H-funktionellen Reaktanten (2) und Phosgen (3) zu einem Reaktionsgemisch (50),
A.II) eine mit der Mischzone (1100) verbundene Reaktionszone (1200) zur Umsetzung des in A.I) erhaltenen Reaktionsgemisches (50) unter Ausbildung einer Flüssigphase (60) enthaltend das chemische Produkt (1) sowie Phosgen (3) und eines Phosgen-haltigen Prozessabgasstroms (70);
B) eine mit der Reaktionsstrecke (1000) verbundene Aufarbeitungsstrecke (2000) aufweisend:
B.I) eine Trenneinheit (2100-2500) zur Trennung der in A.II) erhaltenen Flüssigphase (60) in einen Phosgen-haltigen Prozessabgasstrom (170) und in eine Flüssigphase (100) enthaltend das chemische Produkt (1),
C) eine Abgasaufarbeitungsstrecke (3000) geeignet zur Aufarbeitung während der Herstellung des chemischen Produkts (1) und während der Produktionsunterbrechung anfallender Phosgen-haltiger Abgasströme, aufweisend eine erste Phosgenzersetzungseinrichtung (3011) und eine zweite Phosgenzersetzungseinrichtung (3012), wobei die erste und die zweite Phosgenzersetzungseinrichtung getrennt voneinander mit Phosgen-haltigen Abgasströmen anströmbar sind;
D) eine Verbrennungseinrichtung (6000) geeignet zur Verbrennung des in Abgasaufarbeitungsstrecke (3000) anfallenden aufgearbeiteten Abgases, wobei die Verbrennungseinrichtung (6000) so mit der Abgasaufarbeitungsstrecke (3000) verbunden ist, dass die in der ersten Phosgenzersetzungseinrichtung (3011) und in der zweiten Phosgenzersetzungseinrichtung (3012) anfallenden Abgasströme der Verbrennungseinrichtung (6000) getrennt voneinander zugeführt werden
wobei die Produktionsanlage derart ausgestaltet ist, dass bei einer Produktionsunterbrechung durch Abstellen der Zufuhr des H-funktionellen Reaktanten (2) und Außerbetriebnahme mindestens eines Anlagenteils ein Ausgangsstrom dieses mindestens einen nicht außer Betrieb genommenen Anlagenteils
(i) in den jeweiligen Anlagenteil oder
(ii) in einen strömungstechnisch davor oder dahinter liegenden Anlagenteil geführt und von dort, optional über weitere nicht außer Betrieb genommene Anlagenteile, in den ausgehenden Anlagenteil
zurückgeführt und so der jeweilige Anlagenteil in Kreislauffahrweise gebracht werden kann,
wobei die Produktionsanlage ferner derart ausgestaltet ist, dass
• in dem mindestens einen in Kreislauffahrweise gebrachten Anlagenteil anfallendes Prozessabgas in die erste Phosgenzersetzungseinrichtung (3100) und
• in dem mindestens einen außer Betrieb genommenen Anlagenteil anfallendes Sauerstoff-haltiges Abgas (400-X) in die zweite Phosgenzersetzungseinrichtung (3200)
geführt werden kann, ohne dass Prozessabgas und Sauerstoff-haltiges Abgas (400-X) miteinander vermischt werden.

## Claims

1. Method of operating a production plant for preparation of a chemical product (1) by reacting an H-functional reactant (2) with phosgene (3) in the event of a production stoppage, wherein the production plant has the following plant components:
A) a reaction section (1000) suitable for reacting an H-functional reactant (2) with phosgene (3), having:
A.I) a mixing zone (1100) for mixing the H-functional reactant (2) and phosgene (3) to give a reaction mixture (50),
A.II) a reaction zone (1200) connected to the mixing zone (1100) for reacting the reaction mixture (50) obtained in A.I) to form a liquid phase (60) comprising the chemical product (1) and phosgene (3) and a phosgene-containing process offgas stream (70);
B) a workup section (2000) connected to the reaction section (1000) and having:
B.I) a separation unit (2100-2500) for separating the liquid phase (60) obtained in A.II) into a phosgene-containing process offgas stream (170) and into a liquid phase (100) comprising the chemical product (1);
C) an offgas workup section (3000) suitable for workup of phosgene-containing offgas streams obtained during the preparation of the chemical product (1) and during the production stoppage, comprising a first phosgene breakdown unit (3011) and a second phosgene breakdown unit (3012), wherein the first and second phosgene breakdown unit are configured to receive inflow of phosgene-containing offgas streams independently of one another;
D) an incineration unit (6000) suitable for incineration of the worked-up offgas obtained in offgas workup section (3000);
wherein phosgene (3) is used in a stoichiometric excess relative to all active hydrogen atoms of the H-functional reactant during the preparation of the chemical product (1) in the reaction section (1000), wherein the phosgene-containing process offgas stream (70) obtained from A.II) in the reaction zone (1200) and the phosgene-containing process offgas stream (170) obtained from B.I) in the separation unit (2100-2500), each optionally after passing through further workup steps, are sent to the first phosgene breakdown unit (3011),
wherein furthermore the production of the chemical product (1) is stopped temporarily by switching off the supply of the H-functional reactant (2),
wherein, during this production stoppage,
• at least one plant component of A) and/or B) is shut down and, in at least one of the plant components that have not been shut down, an output stream from this at least one plant component that has not been shut down is conducted
(i) into the respective plant component or
(ii) into an upstream or downstream plant component and thence, optionally via further plant components that have not been shut down, recycled into the original plant component
and hence the respective plant component is put in circulation mode, wherein process offgas is obtained in the at least one plant component put in circulation mode and oxygen-containing offgas (400-X) is obtained in the at least one plant component that has been shut down;
• process offgas from the at least one plant component put in circulation mode is conducted into the first phosgene breakdown unit (3011), wherein the first phosgene breakdown unit (3011) remains in operation even during the production stoppage;
• oxygen-containing offgas (400-X) from the at least one plant component that has been shut down is supplied to the second phosgene breakdown unit (3012), wherein the second phosgene breakdown unit (3012) is in operation at least during the production stoppage, wherein
• process offgas (210-X) that has been freed of phosgene from the first phosgene breakdown unit (3011) and oxygen-containing offgas (410-X) that has been freed of phosgene from the second phosgene breakdown unit (3012) are supplied separately to and combusted in the incineration unit (6000) at spatially separate points.

2. Method according to Claim 1, in which the chemical product (1) is an organic carbonate, especially a polycarbonate.

3. Method according to Claim 1, in which the chemical product (1) is an isocyanate, especially tolylene diisocyanate or a mixture of methylene diphenylene diisocyanate and polymethylene polyphenylene polyisocyanate.

4. Method according to Claim 3, in which the separation unit (2100-2500) from B.I) has
• a distillation apparatus (2100) for separating the liquid stream (60) into a liquid stream (80) comprising solvent and isocyanate and a gaseous process offgas stream (90) comprising phosgene and hydrogen chloride;
• a distillation apparatus (2200) for separating the liquid stream (80) comprising solvent and isocyanate into a process offgas stream (110) comprising solvent and a liquid stream (100) comprising isocyanate;
• a distillation apparatus (2300) for separating the process offgas stream (110) comprising solvent into a liquid stream (120) comprising solvent and a gaseous, phosgene-containing process offgas stream (130).

5. Method according to Claim 4, in which the separation unit (2100-2500) from B.I) also has an absorption apparatus (2500) in which the phosgene-containing process offgas streams (70), (90) and (130) are cleaned by absorption in a solvent (4) to obtain a liquid stream (160) comprising solvent and phosgene and a gaseous process offgas stream (170) comprising hydrogen chloride and solvent, wherein the gaseous phosgene-containing process offgas streams (70) and (90) are first preferably combined and the combined phosgene-containing process offgas stream of (70) and (90) and the phosgene-containing process offgas stream (130) are each condensed and then introduced in liquid form into the absorption apparatus (2500).

6. Method according to any of Claims 3 to 5, especially according to Claim 5, in which the workup section (2000) from B) has, in addition to the separation unit (2100-2500) from B.I),
B.II) a separation unit (2600) for separation of hydrogen chloride from the phosgene-containing process offgas stream (170)
in which the phosgene-containing process offgas stream (170) is depleted of hydrogen chloride, wherein, preferably after passage through a vapor condenser (2630), a gaseous phosgene-containing process offgas stream (200) comprising solvent and any gaseous secondary components is obtained, wherein the phosgene-containing process offgas stream (200) is sent to the first phosgene breakdown unit (3011).

7. Method according to Claim 6, in which the separation of the hydrogen chloride in the separation unit (2600) is performed by absorption of hydrogen chloride in water or hydrochloric acid at a concentration in the range from 0.50% by mass to 15.0% by mass, based on the total mass of the hydrochloric acid, to obtain a hydrochloric acid-containing stream (190) in addition to the phosgene-containing process offgas stream (200) comprising solvent and any gaseous secondary components.

8. Method according to any of Claims 3 to 7, in which the workup section (2000) from B) has, in addition to the separation unit (2100-2500) from B.I.) and, if present, in addition to the separation unit (2600) from B.II),
B.III) a distillation unit (2400) for workup of the liquid phase (100) comprising the isocyanate,
wherein the distillation unit (2400) is optionally preceded upstream by a unit for removing polymeric isocyanate fractions (2410).

9. Method according to Claim 8, having the unit for removing polymeric isocyanate fractions (2410), in which, during the production stoppage,
• a first circulation mode proceeding from the top of the distillation apparatus (2200) via the distillation apparatus (2300) back into the distillation apparatus (2200);
• a second circulation mode proceeding from the bottom of the distillation apparatus (2200) via the unit for removing polymeric isocyanate fractions (2410) back into the distillation apparatus (2200);
• a third circulation mode proceeding from the distillation apparatus (2100) and back into it, and
• a fourth circulation mode proceeding from the separation unit (2600) for separating hydrogen chloride and back into it
are established, wherein the reaction section (1000) from A) is shut down.

10. Method according to any of the preceding claims, in which the production plant has a phosgene preparation section (0) comprising an apparatus (4000) for preparation of phosgene from carbon monoxide (300) and chlorine (310), wherein the preparation of phosgene is shut down when production is stopped, optionally with a time delay after the supply of the H-functional reactant (2) has been switched off.

11. Method according to any of the preceding claims, in which the plant components of the workup section (2000) from B) are operated at least partially at reduced pressure relative to ambient pressure, wherein the reduced pressure is generated by vacuum generation plants in which process offgas streams that are supplied to the first phosgene breakdown unit (3011) are obtained.

12. Method according to Claim 11, in which the first phosgene breakdown unit (3011) has at least two separately operated phosgene breakdown plant components (3011-1, 3011-2), wherein, during the preparation of the chemical product (1), one of these two phosgene breakdown plant components (3011-1) is supplied solely with the process offgas streams from the vacuum generation plants, while the other phosgene breakdown plant component (3011-2) is supplied with all other process offgas streams, wherein, during a production stoppage, the vacuum generation plants are shut down and remain connected to the phosgene breakdown plant component (3011-1).

13. Method according to any of the preceding claims, in which, during the production of the chemical product, oxygen-containing offgas streams (410-X) are obtained, wherein the second phosgene breakdown unit (3012) for oxygen-containing offgas streams is operated during the production of the chemical product (1) as well and it is supplied with the oxygen-containing offgas streams (410-X).

14. Method according to Claim 13, in which the second phosgene breakdown unit (3012) comprises at least two phosgene breakdown plant components (3012-1, 3012-2) connected in parallel, wherein one of the phosgene breakdown plant components (3012-1) is supplied solely with the oxygen-containing offgas streams (410-X) obtained during the production of the chemical product, while the other phosgene breakdown plant component (3012-2) is supplied with the oxygen-rich offgas streams (400-X) obtained in the at least one plant component that has been shut down in the event of a production stoppage.

15. Production plant for preparation of a chemical product (1) by reacting an H-functional reactant (2) with phosgene (3), having the following plant components:
A) a reaction section (1000) suitable for reacting an H-functional reactant (2) with phosgene (3), having:
A.I)a mixing zone (1100) for mixing the H-functional reactant (2) and phosgene (3) to give a reaction mixture (50),
A.II) a reaction zone (1200) connected to the mixing zone (1100) for reacting the reaction mixture (50) obtained in A.I) to form a liquid phase (60) comprising the chemical product (1) and phosgene (3) and a phosgene-containing process offgas stream (70);
B) a workup section (2000) connected to the reaction section (1000) and having:
B.I)a separation unit (2100-2500) for separating the liquid phase (60) obtained in A.II) into a phosgene-containing process offgas stream (170) and into a liquid phase (100) comprising the chemical product (1),
C) an offgas workup section (3000) suitable for workup of phosgene-containing offgas streams obtained during the preparation of the chemical product (1) and during the production stoppage, comprising a first phosgene breakdown unit (3011) and a second phosgene breakdown unit (3012), wherein the first and second phosgene breakdown unit are configured to receive inflow of phosgene-containing offgas streams independently of one another;
D) an incineration unit (6000) suitable for incineration of the worked-up offgas obtained in offgas workup section (3000), wherein the incineration unit (6000) is connected to the offgas workup section (3000) in such a way that the offgas streams obtained in the first phosgene breakdown unit (3011) and in the second phosgene breakdown unit (3012) are sent separately to the incineration unit (6000),
wherein the production plant is configured such that, in the event of a production stoppage by switching off the supply of the H-functional reactant (2) and shutting down at least one plant component, an output stream from this at least one plant component that has not been shut down is conducted
(i) into the respective plant component or
(ii) into an upstream or downstream plant component and thence, optionally via further plant components that have not been shut down, recycled into the original plant component
and hence the respective plant component can be put in circulation mode,
wherein the production plant is further configured such that
• process offgas obtained in the at least one plant component put in circulation mode can be conducted into the first phosgene breakdown unit (3100) and
• oxygen-containing offgas (400-X) obtained in the at least one plant component that has been shut down can be conducted into the second phosgene breakdown unit (3200),
without mixing of process offgas and oxygen-containing offgas (400-X) with one another.

## Revendications

1. Procédé pour l'exploitation d'une installation de production pour la préparation d'un produit chimique (1) par transformation d'un réactant (2) fonctionnalisé par H avec du phosgène (3) lors d'une interruption de production, installation de production présentant les parties d'installation suivantes :
A) un trajet de réaction (1000) approprié pour la transformation d'un réactant (2) fonctionnalisé par H avec du phosgène (3), présentant :
A.I) une zone de mélange (1100) pour le mélange du réactant (2) fonctionnalisé par H et du phosgène (3) pour donner un mélange de réaction (50),
A.II) une zone de réaction (1200) reliée avec la zone de mélange (1100) pour la transformation du mélange de réaction (50) obtenu en A.I) avec formation d'une phase liquide (60) contenant le produit chimique (1) ainsi que du phosgène (3) et un flux de gaz d'échappement de procédé (70) contenant du phosgène ;
B) un trajet de traitement (2000) relié avec le trajet de réaction (1000) présentant :
B.I) une unité de séparation (2100 à 2500) pour la séparation de la phase liquide (60) obtenue en A.II) en un flux de gaz d'échappement de procédé (170) contenant du phosgène et en une phase liquide (100) contenant le produit chimique (1) ;
C) un trajet de traitement de gaz d'échappement (3000) approprié pour le traitement de flux de gaz d'échappement contenant du phosgène produits pendant la préparation du produit chimique (1) et pendant l'interruption de production, présentant un premier dispositif de décomposition de phosgène (3011) et un deuxième dispositif de décomposition de phosgène (3012), le premier et le deuxième dispositif de décomposition de phosgène pouvant être parcourus de manière séparée l'un de l'autre par des flux de gaz d'échappement contenant du phosgène ;
D) un dispositif de combustion (6000) approprié pour la combustion du gaz d'échappement traité produit dans le trajet de traitement de gaz d'échappement (3000) ;
dans lequel pendant la préparation du produit chimique (1) dans le trajet de réaction (1000), le phosgène (3) est utilisé en excès stœchiométrique par rapport à tous les atomes d'hydrogène actifs du réactant fonctionnalisé par H, le flux de gaz d'échappement de procédé (70) contenant du phosgène obtenu dans la zone de réaction (1200) de A.II) et le flux de gaz d'échappement de procédé (170) contenant du phosgène produit dans l'unité de séparation (2100 à 2500) de B.I), à chaque fois éventuellement après le déroulement d'autres étapes de traitement, étant alimentés au premier dispositif de décomposition de phosgène (3011),
la production du produit chimique (1) étant en outre temporairement interrompue par l'arrêt de l'alimentation du réactant fonctionnalisé par H (2), dans lequel pendant cette interruption de production
• au moins une partie d'installation parmi A) et/ou B) est mise hors service et dans au moins l'une des parties d'installation non mises hors service, un flux sortant de cette au moins une partie d'installation non mise hors service est conduit
(i) dans la partie d'installation respective ou
(ii) dans une partie d'installation située fluidiquement avant ou après et de là, éventuellement par l'intermédiaire d'autres parties d'installation non mises hors service, est recyclé dans la partie d'installation sortante
et ainsi la partie d'installation respective est mise en fonctionnement en boucle, dans lequel un gaz d'échappement de procédé est produit dans l'au moins une partie d'installation mise en fonctionnement en boucle et un gaz d'échappement contenant de l'oxygène (400-X) est produit dans l'au moins une partie d'installation mise hors service ;
• un gaz d'échappement de procédé provenant de l'au moins une partie d'installation mise en fonctionnement en boucle est conduit dans le premier dispositif de décomposition de phosgène (3011), le premier dispositif de décomposition de phosgène (3011) restant en fonctionnement également pendant l'interruption de production ;
• un gaz d'échappement contenant de l'oxygène (400-X) provenant de l'au moins une partie d'installation mise hors service est alimenté au deuxième dispositif de décomposition de phosgène (3012), le deuxième dispositif de décomposition de phosgène (3012) étant en fonctionnement au moins pendant l'interruption de production,
• un gaz d'échappement de procédé (210-X) sans phosgène provenant du premier dispositif de décomposition de phosgène (3011) et un gaz d'échappement contenant de l'oxygène (410-X) sans phosgène provenant du deuxième dispositif de décomposition de phosgène (3012) étant alimentés de manière séparée l'un de l'autre au dispositif de combustion (6000) en des emplacements séparés l'un de l'autre et y étant brûlés.

2. Procédé selon la revendication 1, dans lequel le produit chimique (1) est un carbonate organique, en particulier un polycarbonate.

3. Procédé selon la revendication 1, dans lequel le produit chimique (1) est un isocyanate, en particulier un diisocyanate de toluylène ou un mélange de diisocyanate de méthylènediphénylène et de polyisocyanate de polyméthylènepolyphénylène.

4. Procédé selon la revendication 3, dans lequel l'unité de séparation (2100 à 2500) de B.I) présente
• un dispositif de distillation (2100) pour la séparation du flux liquide (60) en un flux liquide (80) contenant du solvant et un isocyanate et un flux de gaz d'échappement de procédé gazeux (90) contenant du phosgène et du chlorure d'hydrogène ;
• un dispositif de distillation (2200) pour la séparation du flux liquide (80) contenant du solvant et un isocyanate en un flux de gaz d'échappement de procédé (110) contenant du solvant et un flux liquide (100) contenant un isocyanate ;
• un dispositif de distillation (2300) pour la séparation du flux de gaz d'échappement de procédé (110) contenant du solvant en un flux liquide (120) contenant du solvant et un flux de gaz d'échappement de procédé gazeux (130) contenant du phosgène.

5. Procédé selon la revendication 4, dans lequel l'unité de séparation (2100 à 2500) de B.I) présente en outre un dispositif d'absorption (2500), dans lequel les flux de gaz d'échappement de procédé (70), (90) et (130) contenant du phosgène sont purifiés par absorption dans un solvant (4) avec obtention d'un flux liquide (160) contenant du solvant et du phosgène et d'un flux de gaz d'échappement de procédé gazeux (170) contenant du chlorure d'hydrogène et du solvant, préférablement les flux de gaz d'échappement de procédé gazeux (70) et (90) contenant du phosgène étant d'abord réunis et le flux de gaz d'échappement de procédé contenant du phosgène réuni de (70) et (90) ainsi que le flux de gaz d'échappement de procédé (130) contenant du phosgène étant à chaque fois condensés et ensuite introduits sous forme liquide dans le dispositif d'absorption (2500).

6. Procédé selon l'une quelconque des revendications 3 à 5, en particulier selon la revendication 5, dans lequel le trajet de traitement (2000) de B) présente en plus de l'unité de séparation (2100 à 2500) de B.I),
B.II) une unité de séparation (2600) pour la séparation de chlorure d'hydrogène du flux de gaz d'échappement de procédé (170) contenant du phosgène,
dans laquelle le flux de gaz d'échappement de procédé (170) contenant du phosgène est appauvri en chlorure d'hydrogène, dans lequel, préférablement après passage d'un condensateur de vapeur (2630), un flux de gaz d'échappement de procédé gazeux (200) contenant du phosgène, contenant du solvant et éventuellement des composants secondaires gazeux est obtenu, le flux de gaz d'échappement de procédé (200) contenant du phosgène étant alimenté au premier dispositif de décomposition de phosgène (3011) .

7. Procédé selon la revendication 6, dans lequel la séparation du chlorure d'hydrogène dans l'unité de séparation (2600) est mise en œuvre par absorption de chlorure d'hydrogène dans de l'eau ou de l'acide chlorhydrique d'une concentration dans la plage de 0,50 % en masse à 15,0 % en masse, par rapport à la masse totale de l'acide chlorhydrique, un flux (190) contenant de l'acide chlorhydrique étant obtenu, outre le flux de gaz d'échappement de procédé (200) contenant du phosgène, contenant du solvant et éventuellement des composants secondaires gazeux.

8. Procédé selon l'une quelconque des revendications 3 à 7, dans lequel le trajet de traitement (2000) de B) présente en plus de l'unité de séparation (2100 à 2500) de B.I) et, si elle est présente, en plus de l'unité de séparation (2600) de B.II),
B.III) une unité de distillation (2400) pour le traitement de la phase liquide (100) contenant l'isocyanate, éventuellement un dispositif pour la séparation de fractions d'isocyanate polymériques (2410) étant montée en amont de l'unité de distillation (2400).

9. Procédé selon la revendication 8, présentant le dispositif pour la séparation de fractions d'isocyanate polymériques (2410), dans lequel pendant l'interruption de production
• un premier fonctionnement en boucle est établi à partir de la tête du dispositif de distillation (2200) par l'intermédiaire du dispositif de distillation (2300) avant de revenir vers le dispositif de distillation (2200) ;
• un deuxième fonctionnement en boucle est établi à partir du fond du dispositif de distillation (2200) par l'intermédiaire du dispositif pour la séparation de fractions d'isocyanate polymériques (2410) avant de revenir vers le dispositif de distillation (2200) ;
• un troisième fonctionnement en boucle est établi en partant du dispositif de distillation (2100) et en revenant vers celui-ci, et
• un quatrième fonctionnement en boucle est établi en partant de l'unité de séparation (2600) pour la séparation de chlorure d'hydrogène et en revenant vers celle-ci,
le trajet de réaction (1000) de A) étant mis hors service.

10. Procédé selon l'une quelconque des revendications précédentes, dans lequel l'installation de production dispose d'un trajet de préparation de phosgène (0) comprenant un dispositif (4000) pour la préparation de phosgène à partir de monoxyde de carbone (300) et de chlore (310), la préparation de phosgène étant stoppée lors de l'interruption de production, éventuellement de manière décalée dans le temps après l'arrêt de l'alimentation du réactant fonctionnalisé par H (2).

11. Procédé selon l'une quelconque des revendications précédentes, dans lequel les parties d'installation du trajet de traitement (2000) de B) fonctionnent au moins partiellement sous pression réduite par rapport à la pression atmosphérique, la pression réduite étant générée par des installations de génération de vide, dans lesquelles des flux de gaz d'échappement de procédé sont produits, lesquels sont alimentés au premier dispositif de décomposition de phosgène (3011).

12. Procédé selon la revendication 11, dans lequel le premier dispositif de décomposition de phosgène (3011) présente au moins deux installations partielles (3011-1, 3011-2) de décomposition de phosgène fonctionnant de manière séparée l'une de l'autre, dans lequel pendant la préparation du produit chimique (1), seulement les flux de gaz d'échappement de procédé provenant des installations de génération de vide sont alimentés à l'une de ces deux installations partielles (3011-1) de décomposition de phosgène, alors que tous les autres flux de gaz d'échappement de procédé sont alimentés à l'autre installation partielle (3011-2) de décomposition de phosgène, dans lequel pendant une interruption de production les installations de génération de vide sont mises hors service et restent reliées à l'installation partielle (3011-1) de décomposition de phosgène.

13. Procédé selon l'une quelconque des revendications précédentes, dans lequel pendant la production du produit chimique des flux de gaz d'échappement contenant de l'oxygène (410-X) sont produits, le deuxième dispositif (3012) de décomposition de phosgène pour des flux de gaz d'échappement contenant de l'oxygène fonctionnant également pendant la production du produit chimique (1) et des flux de gaz d'échappement contenant de l'oxygène (410-X) sont alimentés à celui-ci.

14. Procédé selon la revendication 13, dans lequel le deuxième dispositif (3012) de décomposition de phosgène comprend au moins deux installations partielles (3012-1, 3012-2) de décomposition de phosgène montées en parallèle, seulement les flux de gaz d'échappement contenant de l'oxygène (410-X) produits pendant la production du produit chimique étant alimentés à l'une des installations partielles (3012-1) de décomposition de phosgène, alors que les flux de gaz d'échappement riches en oxygène (400-X) produits lors d'une interruption de production dans l'au moins une partie d'installation mise hors service sont alimentés à l'autre installation partielle (3012-2) de décomposition de phosgène.

15. Installation de production pour la préparation d'un produit chimique (1) par transformation d'un réactant (2) fonctionnalisé par H avec du phosgène (3), présentant les parties d'installation suivantes :
A) un trajet de réaction (1000) approprié pour la transformation d'un réactant (2) fonctionnalisé par H avec du phosgène (3), présentant :
A.I) une zone de mélange (1100) pour le mélange du réactant (2) fonctionnalisé par H et du phosgène (3) pour donner un mélange de réaction (50),
A.II) une zone de réaction (1200) reliée avec la zone de mélange (1100) pour la transformation du mélange de réaction (50) obtenu en A.I) avec formation d'une phase liquide (60) contenant le produit chimique (1) ainsi que du phosgène (3) et un flux de gaz d'échappement de procédé (70) contenant du phosgène ;
B) un trajet de traitement (2000) relié avec le trajet de réaction (1000) présentant :
B.I) une unité de séparation (2100 à 2500) pour la séparation de la phase liquide (60) obtenue en A.II) en un flux de gaz d'échappement de procédé (170) contenant du phosgène et en une phase liquide (100) contenant le produit chimique (1) ;
C) un trajet de traitement de gaz d'échappement (3000) approprié pour le traitement de flux de gaz d'échappement contenant du phosgène produits pendant la préparation du produit chimique (1) et pendant l'interruption de production, présentant un premier dispositif de décomposition de phosgène (3011) et un deuxième dispositif de décomposition de phosgène (3012), le premier et le deuxième dispositif de décomposition de phosgène pouvant être parcourus de manière séparée l'un de l'autre par des flux de gaz d'échappement contenant du phosgène ;
D) un dispositif de combustion (6000) approprié pour la combustion du gaz d'échappement traité produit dans le trajet de traitement de gaz d'échappement (3000), le dispositif de combustion (6000) étant relié de telle manière avec le trajet de traitement de gaz d'échappement (3000) que les flux de gaz d'échappement produits dans le premier dispositif de décomposition de phosgène (3011) et dans le deuxième dispositif de décomposition de phosgène (3012) sont alimentés de manière séparée l'un de l'autre au dispositif de combustion (6000),
l'installation de production étant conçue de telle manière que, lors d'une interruption de production, par l'arrêt de l'alimentation du réactant fonctionnalisé par H (2) et la mise hors service d'au moins une partie d'installation, un flux sortant de cette au moins une partie d'installation non mise hors service est conduit
(i) dans la partie d'installation respective ou
(ii) dans une partie d'installation située fluidiquement avant ou après et de là, éventuellement par l'intermédiaire d'autres parties d'installation non mises hors service, est recyclé dans la partie d'installation sortante
et ainsi la partie d'installation respective peut être mise en fonctionnement en boucle, l'installation de production étant en outre conçue de telle manière que
• un gaz d'échappement de procédé produit dans l'au moins une partie d'installation mise en fonctionnement en boucle puisse être conduit dans le premier dispositif (3100) de décomposition de phosgène et
• un gaz d'échappement contenant de l'oxygène (400-X) produit dans l'au moins une partie d'installation mise hors service puisse être conduit dans le deuxième dispositif (3200) de décomposition de phosgène,
sans qu'un gaz d'échappement de procédé et un gaz d'échappement contenant de l'oxygène (400-X) ne soit mélangés l'un avec l'autre.
